# EUROPEAN PATENT APPLICATION

(11) **EP 4 488 924 A1**
(43) Date of publication of application: **08.01.2025**
(21) Application number: 22928719.8
(22) Date of filing: 28.02.2022
(51) Int. Cl.: G06T 1/00, A61B 5/1171

(54) **INFORMATION PROCESSING DEVICE, AND INFORMATION PROCESSING SYSTEM**

(71) Applicant: NEC Corporation, 108-8001 Tokyo (JP)
(72) Inventor: OGATSU, Toshinobu, Tokyo 108-8001 (JP)
(74) Representative: Betten & Resch
(86) International application number: PCT/JP2022/008174
(87) International publication number: WO 2023/162193

(57) **Abstract**

An information processing apparatus includes: a first imaging unit that is configured to image a target; a second imaging unit that is configured to image the target; a lighting unit that is configured to emit illumination light; a reflection unit that reflects the illumination light emitted by the lighting unit toward the target; and a drive unit that is disposed at a different position from a position where the lighting unit is disposed, and that drives each of the first imaging unit, the second imaging unit, and the reflection unit.

## Description

### Technical Field

This disclosure relates to, for example, technical fields of an information processing system that is configured to authenticate a target, and an information processing apparatus that is available to the information processing system.

### Background Art

Patent Literature 1 describes an example of an information processing system that is configured to authenticate a target by using an iris of the target, and an information processing apparatus that is available to the information processing system.

In addition, as prior art literatures related to this disclosure, Patent Literatures 2 to 9 are cited.

### Citation List

### Patent Literature

Patent Literature 1: JP2002-122899A
Patent Literature 2: International Publication No. WO2018/038158 pamphlet
Patent Literature 3: JP2007-328571A
Patent Literature 4: JP2002-236666A
Patent Literature 5: International Publication No. WO2016/144019 pamphlet
Patent Literature 6: EP Application Publication No. 3265958 description
Patent Literature 7: US Patent Application Publication No. 2019/0026576A1
Patent Literature 8: US Patent Application Publication No. 2003/0169334A1
Patent Literature 9: International Publication No. WO2006/061833 pamphlet

### Technical Problem

It is an example object of this disclosure to provide an information processing apparatus and an information processing system that are intended to improve the techniques/technologies described in Citation List.

### Solution to Problem

An information processing apparatus according to an example aspect of this disclosure includes: a first imaging unit that is configured to image a target; a second imaging unit that is configured to image the target; a lighting unit that is configured to emit illumination light; a reflection unit that reflects the illumination light emitted by the lighting unit toward the target; and a drive unit that is disposed at a different position from a position where the lighting unit is disposed, and that drives each of the first imaging unit, the second imaging unit, and the reflection unit.

An information processing system according to an example aspect of this disclosure includes: an information processing apparatus; and an authentication apparatus, wherein the information processing apparatus includes: a first imaging unit that is configured to generate a first image by imaging a target; a second imaging unit that is configured to generate a second image by imaging the target; a lighting unit that is configured to emit illumination light; a reflection unit that reflects the illumination light emitted by the lighting unit toward the target; and a drive unit that is disposed at a different position from a position where the lighting unit is disposed, and that drives each of the first imaging unit, the second imaging unit, and the reflection unit, and the authentication apparatus includes an authenticate unit that authenticates the target by using at least one of the first image and the second image.

### Brief Description of Drawings

[FIG. 1] FIG. 1 is a block diagram illustrating a configuration of an information processing system in a first example embodiment.
[FIG. 2] FIG. 2 is a block diagram illustrating an overall configuration of an information processing system in a second example embodiment.
[FIG. 3] FIG. 3 is a block diagram illustrating a configuration of an imaging unit provided in the information processing system in the second example embodiment.
[FIG. 4] FIG. 4 is a perspective view illustrating an appearance of the imaging unit in the second example embodiment.
[FIG. 5] FIG. 5 is a perspective view illustrating the appearance of the imaging unit in the second example embodiment.
[FIG. 6] FIG. 6 is a cross-sectional view illustrating an arrangement aspect of a face camera, an iris camera, a LED unit, a reflecting mirror, and a drive motor in the second example embodiment.
[FIG. 7] FIG. 7 is a cross-sectional view illustrating an arrangement aspect of the face camera, the iris camera, the LED unit, the reflecting mirror, and the drive motor in the second example embodiment, in a state of separating them from each other.
[FIG. 8] FIG. 8 is a block diagram illustrating a configuration of an authentication servers in the second example embodiment.
[FIG. 9] FIG. 9 is a flowchart illustrating a flow of an imaging operation performed by the imaging unit and an authentication operation performed by the authentication server.
[FIG. 10] FIG. 10 is a block diagram illustrating a configuration of an imaging unit in a third example embodiment.
[FIG. 11] FIG. 11 is a flowchart illustrating a flow of an operation of changing intensity of illumination light in the third example embodiment.
[FIG. 12] FIG. 12 is a cross-sectional view illustrating a rotation angle of a camera cover.
[FIG. 13] FIG. 13 is a graph illustrating a relation between the rotation angle and the intensity of the illumination light.
[FIG. 14] FIG. 14 is a cross-sectional view illustrating the illumination light passing through an optical member that filles an opening of a housing.
[FIG. 15] FIG. 15 is a block diagram illustrating a configuration of an imaging unit in a fourth example embodiment.
[FIG. 16] FIG. 16 is a flowchart illustrating a flow of an operation of changing luminance of an iris image in a fourth example embodiment.
[FIG. 17] FIG. 17 is a graph illustrating a relation between the rotation angle and a change amount of the luminance of the iris image.
[FIG. 18] FIG. 18 is a block diagram illustrating a configuration of an imaging unit in a fifth example embodiment.
[FIG. 19] FIG. 19 is a cross-sectional view illustrating respective rotation angles of the face camera, the iris camera, and the reflecting mirror;
[FIG. 20] FIG. 20 is a block diagram illustrating a configuration of an imaging unit in a sixth example embodiment.
[FIG. 21] FIG. 21 is a flowchart illustrating a flow of a body temperature determination operation in the sixth example embodiment.
[FIG. 22] FIG. 22 is a flowchart illustrating a flow of an imaging operation and an authentication operation in a seventh example embodiment.

### Description of Example Embodiments

Hereinafter, with reference to the drawings, an information processing apparatus and an information processing system according to example embodiments will be described.

### (1) First Example embodiment

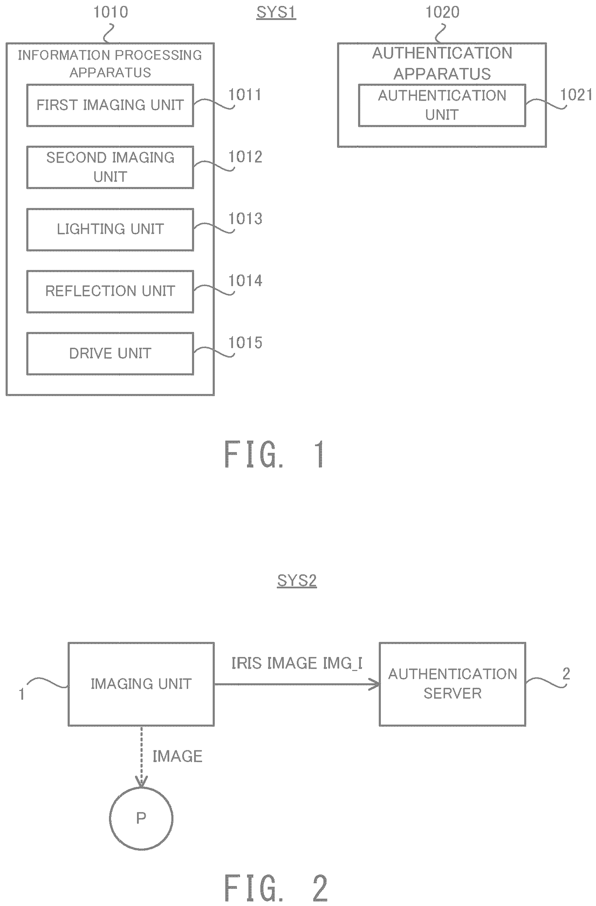

First, an information processing apparatus and an information processing system in a first example embodiment will be described. With reference to FIG. 1, the following describes an information processing system SYS1 to which the information processing system and the information processing apparatus in the first example embodiment are applied. FIG. 1 is a block diagram illustrating a configuration of the information processing system SYS1 in the first example embodiment.

As illustrated in FIG. 1, the information processing system SYS1 includes an information processing apparatus 1010 and an authentication apparatus 1020. The information processing apparatus 1010 includes: a first imaging unit 1011 that is a specific example of the "first imaging unit" described in Supplementary Note later; a second imaging unit 1012 that is a specific example of the "second imaging unit" described in Supplementary Note later; a lighting unit 1013 that is a specific example of the "lighting unit" described in Supplementary Note later; a reflection unit 1014 that is a specific example of the "reflection unit" described in Supplementary Note later, and a drive unit 1015 that is a specific example of the "drive unit" described in Supplementary Note later. The authentication apparatus 1020 includes an authentication unit 1021 that is a specific example of the "authentication unit" described in Supplementary Note later. The information processing apparatus 1010 may be referred to as an imaging apparatus.

The first imaging unit 1011 is configured to image a target. The first imaging unit 1011 may be configured to generate a first image by imaging the target. The second imaging unit 1012 is also configured to image the target. The second imaging unit 1012 may be configured to generate a second image by imaging the target. The first imaging unit 1011 captures a first part of the target, and the second imaging unit 1012 may capture a second part of the target. The first part of the target imaged by the first imaging unit 1011 may be typically different from the second part of the target imaged by the second imaging unit 1012. The first part of the target imaged by the first imaging unit 1011 may include the second part of the target imaged by the second imaging unit 1012. In other words, the second part of the target imaged by the second imaging unit 1012 may be a part included in the first part of the target imaged by the first imaging unit 1011. The first part of the target imaged by the first imaging unit 1011, however, may be the same as the second part of the target imaged by the second imaging unit 1012. Note that the target is typically a person; however, the target is not limited to a person.

The lighting unit 1013 emits illumination light. The reflection unit 1014 reflects the illumination light emitted by the lighting unit 1013, toward the target. Consequently, the illumination light reflected by the reflection unit 1014 is illuminated/applied to the target. That is, the lighting unit 1013 illuminates the target with the illumination light through the reflection unit 1014.

The lighting unit 1013 may illuminate the target with the illumination light in at least a part of a period when the first imaging unit 1011 images the target. The first imaging unit 1011 may image the target illuminated with the illumination light. In this case, return light (e.g., at least one of reflected light and the scattered light) of the illumination light illuminated/applied to the target may enter the first imaging unit 1011. The first imaging unit 1011 may image the target by receiving the return light of the illumination light from the target.

The lighting unit 1013 may illuminate the target with the illumination light in at least a part of a period when the second imaging unit 1012 images the target. The second imaging unit 1012 may image the target illuminated with the illumination light. In this case, return light (e.g., at least one of reflected light and the scattered light) of the illumination light illuminated/applied to the target may enter the second imaging unit 1012. The second imaging unit 1012 may image the target by receiving the return light of the illumination light from the target.

The drive unit 1015 is disposed at a different position from a position where the lighting unit 1013 is disposed. The drive unit 1015 drives each of the first imaging unit 1011, the second imaging unit 1012, and the reflection unit 1014. That is, the drive unit 1015 moves each of the first imaging unit 1011, the second imaging unit 1012, and the reflection unit 1014. On the other hand, the drive unit 1015 may not drive the lighting unit 1013. That is, the drive unit 1015 may not move the lighting unit 1013. In this case, the lighting unit 1013 may be fixed.

The authentication unit 1021 authenticates the target. Therefore, each of the first imaging unit 1011 and the second imaging unit 1012 images the target to be authenticated by the authentication unit 1021. The authentication unit 1021 authenticates the target by using at least one of a first image generated by the first imaging unit 1011 imaging the target and a second image generated by the second imaging unit 1012 imaging the target. As an example, when the first imaging unit 1011 generates a face image as the first image by imaging a face of the target, the authentication unit 1021 may perform face authentication of authenticating the target by using the face image. As another example, when the second imaging unit 1012 generates an iris image as the second image by imaging an iris of the target, the authentication unit 1021 may perform iris authentication of authenticating the target by using the iris image.

In the information processing apparatus 1010 in the first example embodiment, the drive unit 1015 may drive each of the first imaging unit 1011, the second imaging unit 1012, and the reflection unit 1014, but may not drive the lighting unit 1013. As a result, in the first example embodiment, as compared with a first comparative example in which the lighting unit 1013 is driven with each of the first imaging unit 1011, the second imaging unit 1012, and the reflection unit 1014, wires connected to the lighting unit 1013 are less likely to be disconnected due to the drive of each of the first imaging unit 1011, the second imaging unit 1012, and the reflection unit 1014. Therefore, the information processing apparatus 1010 in the first example embodiment is capable of solving a first technical problem that the wires connected to the lighting unit 1013 may be disconnected.

In addition, in the information processing apparatus 1010 in the first example embodiment, the lighting unit 1013 may not be driven with each of the first imaging unit 1011, the second imaging unit 1012, and the reflection unit 1014, which are driven by the drive unit 1015. Therefore, in the first example embodiment, as compared with the first comparative example, the lighting unit 1013 may be disposed at a position away from each of the first imaging unit 1011, the second imaging unit 1012, and the reflection unit 1014. As a result, in the first example embodiment, as compared with the first comparative example, a heat generated in the lighting unit 1013 due to the emission of the illumination light is less likely to be transmitted to each of the first imaging unit 1011, the second imaging unit 1012, and the reflection unit 1014. Therefore, in the first example embodiment, as compared with the first comparative example, an influence on each of the first imaging unit 1011, the second imaging unit 1012, and the reflection unit 1014, which is received from the heat of the lighting unit 1013, is reduced. Accordingly, the information processing apparatus 1010 in the first example embodiment is capable of solving a second technical problem that each of the first imaging unit 1011, the second imaging unit 1012, and the reflection unit 1014 may not operate normally due to the influence of the heat of the lighting unit 1013.

The information processing apparatus 1010 may include another drive unit that drives the lighting unit 1013. In this case, the other drive unit may drive the lighting unit 1013, in accordance with the drive of the reflection unit 1014 by the drive unit 1015. For example, the other drive unit may drive the lighting unit 1013 in the same direction as a direction in which the drive unit 1015 drives the reflection unit 1014. As an example, the other drive unit may drive the lighting unit 1013 upward, when the drive unit 1015 drives the reflection unit 1014 upward.

### (2) Second Example Embodiment

Next, an information processing apparatus and an information processing system in a second example embodiment will be described. With reference to FIG. 2, the following describes an information processing system SYS2 to which the information processing system and the information processing apparatus in the second example embodiment are applied.

### (2-1) Overall Configuration of Information processing System SYS2

First, with reference to FIG. 2, an overall configuration of the information processing system SYS2 in the second example embodiment will be described. FIG. 2 is a block diagram illustrating the overall configuration of the information processing system SYS2 in the second example embodiment.

As illustrated in FIG. 2, the information processing system SYS2 includes an imaging unit 1 and an authentication server 2. The imaging unit 1 may be regarded as a specific example of the "information processing apparatus" described in Supplementary Note later. The imaging unit 1 may be referred to as an information processing apparatus or an information processing unit. The authentication server 2 may be regarded as a specific example of the "authentication apparatus" described in Supplementary Note later. The information processing system SYS2 may be referred to as authentication system.

The imaging unit 1 performs an imaging operation of imaging at least a part of the target. The target may include, for example, a person. The target may include an animal that is different from the person (e.g., at least one of mammals such as dogs and cats, birds such as sparrow, reptiles such as snakes, amphibians such as frogs, and fishes such as goldfish). The target may include an inanimate object. The inanimate object may include a robot that imitates a person or an animal. The following describes a case where the target is a person. Therefore, in the following, the target is referred to as a " target person P".

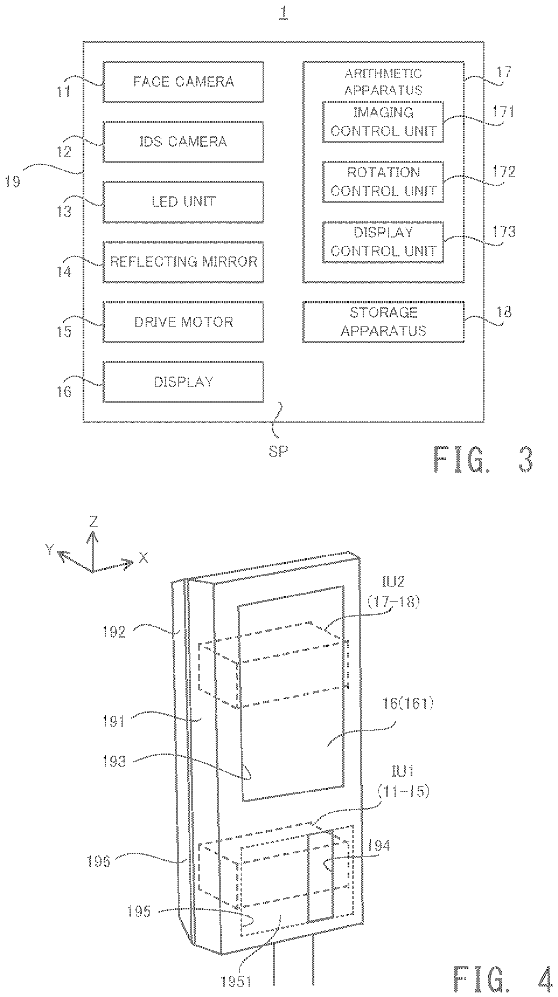

The imaging unit 1 is configured to generate a person image IMG in which at least a part of the target person P is captured, by imaging at least a part of the target person P. Specifically, the imaging unit 1 is configured to generate a face image IMG_F in which a face of the target person P is captured, as the person image IMG, by imaging the face of the target person P with a face camera 11 (see FIG. 3) described later. Furthermore, the imaging unit 1 is configured to generate an iris image IMG_I in which an eye (especially, an iris) of the target person P is captured, as the person image IMG, by imaging the eye (especially, the iris) of the target person P with an iris camera 12 (see FIG. 3) described later.

The iris image IMG_I may include a part of the target person P that is different from the iris. Even in this case, as described in detail later, the target person P is authenticated by using the iris of the target person P captured in the iris image IMG_I. Thus, there is no problem even if a part of the target person P that is different from the iris is captured in the iris image IMG_I. Alternatively, in a case where the iris image IMG_I includes a part of the target person P that is different from the iris, but does not include the iris of the target person P, the imaging unit 1 may generate the iris image IMG_I in which the iris of the target person P is captured, by moving (i.e., driving) the iris camera 12, as described in detail later. Specifically, the imaging unit 1 may rotate (i.e., rotationally move) the iris camera 12 in a tilt direction such that the iris of the target person P is included in an imaging range of the iris camera 12, thereby generating the iris image IMG_I in which the iris of the target person P is captured.

Similarly, the face image IMG_F may include a part of the target person P that is different from the face. Even in this case, as described in detail later, a position of the eye of the target person P is identified by using the face of the target person P captured in the face image IMG_F. Thus, there is no problem even if a part of the target person P that is different from the face is captured in the face image IMG_F. Alternatively, in a case where the face image IMG_F includes a part of the target person P that is different from the face, but does not include the face of the target person P, the imaging unit 1 may generate the face image IMG_F in which the face of the target person P is captured, by moving (i.e., driving) the face camera 11, as described in detail later. Specifically, the imaging unit 1 may rotate (i.e., rotationally drive) the face camera 11 in the tilt direction such that the face of the target person P is included in an imaging range of the face camera 11, thereby generating the face image IMG_F in which the face of the target person P is captured.

The authentication server 2 acquires the person image IMG from the imaging unit 1 and performs an authentication operation for authenticating the target the person P by using the person image IMG. In the second example embodiment, the authentication server 2 acquires the iris image IMG_I from the imaging unit 1 and performs the authentication operation for authenticating the target the person P by using the iris image IMG I. That is, the authentication server 2 performs the authentication operations about the iris authentication. Specifically, the authentication server 2 determines whether or not the target person P captured in the acquired iris image IMG_I is the same as a person registered in advance (hereinafter referred to as a "registered person") based on an iris pattern of the target person P captured in the acquired iris image IMG_I. When it is determined that the target person P captured in the iris image IMG I is the same as the registered person, the authentication of the target person P is determined to be successful. On the other hand, when it is determined that the target person P captured in the iris image IMG_I is not the same as the registered person, the authentication of the target person P is determined to be failed.

Such an information processing system SYS2 may be used, for example, to manage/control entry of the target person P to an entry restricted area where the target person P satisfying a predetermined entry condition is permitted to enter, but the target person P not satisfying the predetermined entry condition is not permitted to enter. In this case, the imaging unit 1 may be disposed at an entrance/exit of the entry restricted area. The iris camera 12 may image the target person P who is about to enter the entry restricted area. Based on the iris image IMG_I, the authentication server 2 may determine whether or not the target person P is the same as the registered person satisfying the entry condition. When the target person P is the same as the registered person satisfying the entry condition, the target person P may be permitted to enter the entry restricted area. For example, the authentication server 2 may set a state of a gate apparatus disposed at the entrance/exit of the entry restricted area, into an opening state in which the gate apparatus does not block the passage of the target person P. On the other hand, when the target person P is not the same as the registered person satisfying the entry condition, the target person P may be prohibited from entering the entry restriction area. For example, the authentication server 2 may set the state of the gate apparatus disposed at the entrance/exit of the entry restricted area, into a closing state in which the gate apparatus prevents the passage of the target person P.

Such an information processing SYS2 may be used, for example, to manage/control a payment for goods or services by the target person P. In this instance, the imaging unit 1 may be disposed at a location (e.g., a checkout counter) where the target person P pays for the goods or services. The iris camera 12 may image the target person P who is about to pay. Based on the iris image IMG_I, the authentication server 2 may determine whether or not the target person P is the same as the registered person with which a settlement method is associated in advance. When the target person P is the same as the registered person, the authentication server 2 may complete the payment by the associated settlement method. On the other hand, when the target person P is not the same as the registered person, the authentication server 2 may not need to complete the payment by the associated settlement method.

### (2-2) Configuration of Imaging Unit 1

Next, with reference to FIG. 3, a configuration of the imaging unit 1 will be described. FIG. 3 is a block diagram illustrating the configuration of the imaging unit 1.

As illustrated in FIG. 3, the imaging unit 1 includes the face camera 11, the iris camera 12, a LED (Light Emitting Diode) unit 13, a reflecting mirror 14, a drive motor 15, a display 16, an arithmetic apparatus 17, and a storage apparatus 18. The face camera 11 is a specific example of the "first imaging unit" described in Supplementary Note later. The iris camera 12 is a specific example of the "second imaging unit" described in Supplementary Note later. The LED unit 13 is a specific example of the "lighting unit" described in Supplementary Note later. The reflecting mirror 14 is a specific example of the "reflection unit" described in Supplementary Note later. The drive motor 15 is a specific example of the "drive unit" described in Supplementary Note later.

The face camera 11 is an information processing apparatus that is configured to image the face of the target person P. The face camera 11 is typically configured to image at least a part of the target person P including the face of the target person P. The face camera 11 is configured to generate the face image IMG_F in which the face of the target person P is captured, by imaging the face of the target person P.

The iris camera 12 is an information processing apparatus that is configured to image at least the eye (especially the iris) of the target person P. The iris camera 12 is typically configured to image at least a part of the target person P including the eye of the target person P. The iris camera 12 is configured to generate the iris image IMG_I in which the eye of the target person P is captured, by imaging the eye (especially, the iris) of the target person P.

The LED unit 13 emits illumination light. The illumination light is light for illuminating the target person P. For example, the illumination light may include light for illuminating the face of the target person P. In this instance, the face camera 11 may image the face of the target person P illuminated with the illumination light by the LED unit 13. For example, the illumination light may include light for illuminating the eye (especially the iris) of the target person P. In this instance, the iris camera 12 may image the eye of the target person P illuminated with the illumination light by the LED unit 13.

The following describes an example in which the illumination light includes light for illuminating the eye of the target person P. That is, the following describes an example in which the LED unit 13 emits the illumination light for illuminating the eye of the target person P. As the illumination light for illuminating the eye, typically, near infrared light is used. This is because the target person P may feel glare, when visible light is irradiated to the eye of the target person P as the illumination light. The near infrared light may include light whose wavelength is included in a wavelength band of near infrared rays. As the illumination light, however, different light from the near infrared light (e.g., visible light) may be used.

The reflecting mirror 14 is an optical element that is configured reflect the illumination light emitted by The LED unit 13, toward the target person P. Therefore, in the second example embodiment, the LED unit 13 illuminates the target person P with the illumination light through the reflecting mirror 14. As described above, since the LED unit 13 illuminates the eye of the target person P with the illumination light, the reflecting mirror 14 reflects the illumination light emitted by The LED unit 13, toward the target person P.

At least a part of the illumination light with which the eye of the target person P is illuminated, enters the iris camera 12 as return light returning from the target person P toward the imaging unit 1. The return light may include reflected light of the illumination light (i.e., light reflected by the eye of the target person P). The return light may include scattered light of the illumination light (i.e., light scattered by the eye of the target person P). The iris camera 12 images the eye of the target person P by receiving the return light. Therefore, the iris camera 12 may include an image sensor that receives the return light. An example includes a CCD (Charge Coupled Device) or a CMOS (Complementary Metal Oxide Semiconductor).

The drive motor 15 is a drive apparatus for driving (i.e., moving) the face camera 11, the iris camera 12, and the reflecting mirror 14 under the control of the arithmetic apparatus 17. In the second example embodiment, the drive motor 15 rotates (i.e., rotationally moves) the face camera 11, the iris camera 12, and the reflecting mirror 14 around a predetermined rotation axis. When the drive motor 15 rotates the face camera 11, the imaging range of the face camera 11 moves. Therefore, the drive motor 15 may rotate the face camera 11 such that the face camera 11 is capable of properly imaging the face of the target person P. When the drive motor 15 rotates the iris camera 12, the imaging range of the iris camera 12 moves. Therefore, the drive motor 15 may rotate the iris camera 12 such that the iris camera 12 is capable of properly imaging the eye of the target person P. When the drive motor 15 rotates the reflecting mirror 14, an irradiation position of the illumination light through the reflecting mirror 14 changes. Therefore, the drive motor 15 may rotate the reflecting mirror 14 such that the illumination light is irradiated to the eye of the target person P.

The display 16 is a display apparatus that is configured to display desired information. For example, the display 16 may be configured to display information about the authentication of the target person P using the iris image IMG_I. The information about the authentication of the target person P may include information about an authentication result of the target person P. The information about the authentication of the target person P may include information of which the target person P who succeeds in the authentication is to be notified (e.g., information of which the target person P who is permitted to enter the entry restricted area described above is to be notified). The information about the authentication of the target person P may include information of which the target person P who fails in the authentication is to be notified (e.g., information about a next operation to be performed by the target person P because the authentication is failed).

The imaging unit 1 may include an arbitrary output apparatus that is configured to output desired information, in addition to or in place of the display 16. For example, the imaging unit 1 may include an audio output apparatus (e.g., a speaker) that is configured to output desired information as audio/sound. For example, the imaging unit 1 may include a paper output apparatus (e.g., a printer) that is configured to output a paper sheet on which desired information is described.

The arithmetic apparatus 17 includes at least one of a CPU (Central Processing Unit), a GPU (Graphics Processing Unit), and a FPGA (Field Programmable Gate Array), for example. The arithmetic apparatus 17 reads a computer program. For example, the arithmetic apparatus 17 may read a computer program stored in the storage apparatus 18. For example, the arithmetic apparatus 17 may read a computer program stored in a computer readable and non-transitory recording medium, by a not-illustrated recording medium reading apparatus provided in the imaging unit 1. The arithmetic apparatus 17 may acquire (i.e., download or read) a computer program from a not-illustrated apparatus disposed outside the imaging unit 1, through a not-illustrated communication apparatus. The arithmetic apparatus 17 executes the read computer program. Consequently, a logical functional block for performing an operation to be performed by the imaging unit 1 (e.g., the imaging operation described above) is realized or implemented in the arithmetic apparatus 17. That is, the arithmetic apparatus 17 is allowed to function as a controller for realizing or implementing the logical functional block for performing an operation (in other words, processing) to be performed by the imaging unit 1.

FIG. 3 illustrates an example of the logical functional block realized or implemented in the arithmetic apparatus 17 to perform the imaging operation. As illustrated in FIG. 3, an imaging control unit 171, a rotation control unit 172, and a display control unit 173 are realized or implemented in the arithmetic apparatus 17. The imaging control unit 171 controls the face camera 11, and the iris camera 12 such that each of the face camera 11 and the iris camera 12 images the target person P. The rotation control unit 172 controls the drive motor 15 to rotate the face camera 11, the iris camera 12, and the reflecting mirror 14. The rotation control unit 172 is a specific example of the "drive control unit" described in Supplementary Note later. The display control unit 173 controls the display 16 to display desired information.

The storage apparatus 18 is configured to store desired data. For example, the storage apparatus 18 may temporarily store a computer program to be executed by the arithmetic apparatus 17. The storage apparatus 18 may temporarily store data that are temporarily used by the arithmetic apparatus 17 when the arithmetic apparatus 17 executes the computer program. The storage apparatus 18 may store data that are stored by the imaging unit 1 for a long time. The storage apparatus 18 may include at least one of a RAM (Random Access Memory), a ROM (Read Only Memory), a hard disk apparatus, a magneto-optical disk apparatus, a SSD (Solid State Drive), and a disk array apparatus. That is, the storage apparatus 18 may include a non-transitory recording medium.

The face camera 11, the iris camera 12, the LED unit 13, the reflecting mirror 14, the drive motor 15, the display 16, the arithmetic apparatus 17, and the storage apparatus 18 are disposed in a housing 19. That is, the face camera 11, the iris camera 12, the LED unit 13, the reflecting mirror 14, the drive motor 15, the display 16, the arithmetic apparatus 17, and the storage apparatus 18 are accommodated in an accommodation space SP inside the housing 19. At least one of the face camera 11, the iris camera 12, the LED unit 13, the reflecting mirror 14, the drive motor 15, the display 16, the arithmetic apparatus 17, and the storage apparatus 18, however, may not be disposed in the housing 19.

The housing 19 may include a front housing 191 and a rear housing 192, as illustrated in FIG. 4. In this case, the accommodation space SP may be formed between the front housing 191 and the rear housing 192 by combining the front housing 191 and the rear housing 192. The face camera 11, the iris camera 12, the LED unit 13, the reflecting mirror 14, the drive motor 15, the display 16, the arithmetic apparatus 17, and the storage apparatus 18 may be disposed in the accommodation space SP.

As illustrated in FIG. 4, the display 16 is accommodated in the housing 19 such that a display surface 161 capable of displaying information is exposed to an outside of the housing 19. Specifically, the display 16 is accommodated in the housing 19 such that the display surface 161 of the display 16 is exposed to the outside of the housing 19 through an opening 193 formed in the housing 19 (e.g., the front housing 191). That is, the display 16 is accommodated in the housing 19 such that the display surface 161 is visually recognizable from the outside of the housing 19 through the opening 193.

On the other hand, the face camera 11, the iris camera 12, the LED unit 13, the reflecting mirror 14, the drive motor 15, the arithmetic apparatus 17, and the storage apparatus 18 may not be accommodated in the housing 19 to be visually recognizable from the outside of the housing 19. That is, the face camera 11, the iris camera 12, the LED unit 13, the reflecting mirror 14, the drive motor 15, the arithmetic apparatus 17, and the storage apparatus 18 may be accommodated in the housing 19 so as not to be visually recognizable from the outside of the housing 19.

As described above, however, the face camera 11 needs to image the face of the target person P. Therefore, an opening 194 that is usable by the face camera 11 to image the face of the target person P may be formed in the housing 19. In this instance, light (e.g., visible light) from the face of the target person P may enter the face camera 11 through the opening 194. The face camera 11 may image the face of the target person P by receiving the light entering the face camera 11 through the opening 194.

Furthermore, as described above, it is necessary that the LED unit 13 illuminates the eye of the target person P with the illumination light and that the iris camera 12 receives the return light from the target person P illuminated with the illumination light, so as to image the eye of the target person P. Therefore, an opening 195 for the LED unit 13 to illuminate the eye of the target person P with the illumination light and for the iris camera 12 to receive the return light from the eye of the target person P, may be formed in the housing 19. In this instance, the LED unit 13 may emit the illumination light toward the eye of the target person P through the opening 195. The iris camera 12 may image the eye of the target person P, by receiving the light entering the iris camera 12 through the opening 195.

As described above, when the illumination light is near infrared light, the opening 195 may be filled with an optical member 1951 that allows the near infrared light to pass through and that absorbs or reflects a part of visible light. The opening 195 may be filled with the optical member 1951 that allows the near infrared light to pass through and that exhibits a desired color to the visible light. As a result, design of external appearance of the housing 19 (i.e., design of external appearance of the imaging unit 1) is improved. Furthermore, since it is hard for the target person P to visually recognize an inner structure of the imaging unit 1 through the opening 195, a line of sight of the target person P is easily guided to the display 16 exposed to the outside of the imaging unit 1.

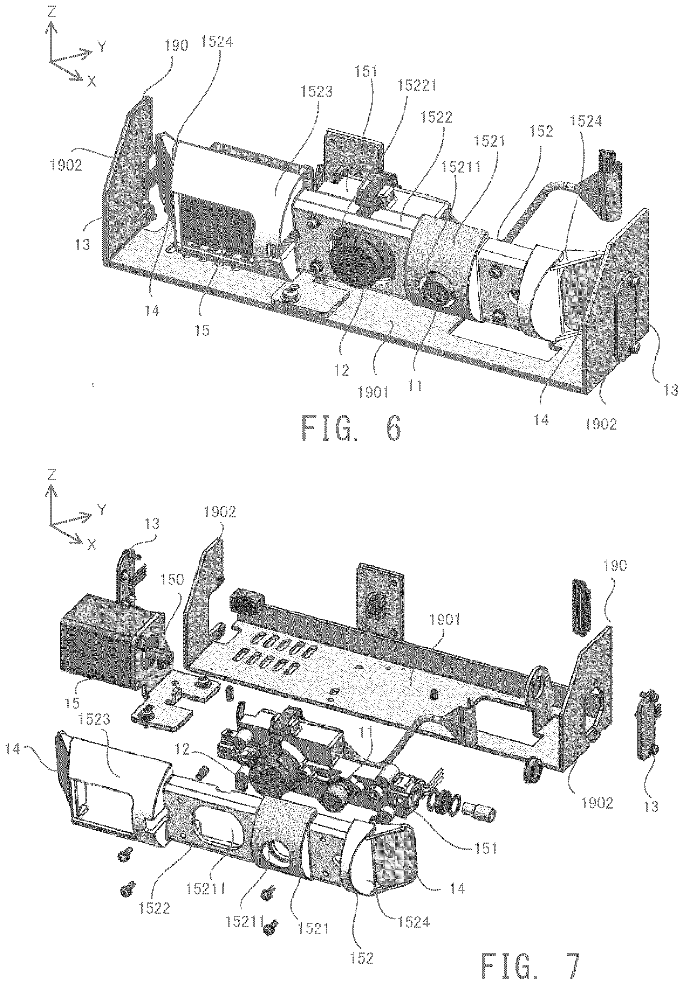

In the second example embodiment, as illustrated in FIG. 4, the face camera 11, the iris camera 12, the LED unit 13, the reflecting mirror 14, and the drive motor 15 may be disposed inside the housing 19 in a form of an integrated unit IU1 in which the face camera 11, the iris camera 12, the LED unit 13, the reflecting mirror 14, and the drive motor 15 are integrated through a common unit base 190 (see FIG. 6 to FIG. 7). As an example, the integrated unit IU1 may be disposed in the vicinity of the openings 194 and 195 in the accommodation space SP inside the housing 19. Furthermore, the arithmetic apparatus 17 and the storage apparatus 18 may be disposed inside the housing 19 in a form of an integrated unit IU2 in which the arithmetic apparatus 17 and the storage apparatus 18 are integrated through a common unit base (not illustrated). As an example, the integrated unit IU2 may be disposed behind the display 16 in the accommodation space SP inside the housing 19. A method of arranging the face camera 11, the iris camera 12, the LED unit 13, the reflecting mirror 14, the drive motor 15, the display 16, the arithmetic apparatus 17, and the storage apparatus 18, however, is not limited to the example illustrated in FIG. 4.

The imaging unit 1 may include a heat radiating member capable of radiating a heat of the accommodation space SP inside the housing 19 to the outside of the housing 19. For example, in the accommodation space SP, there is a possibility that heat is generated from at least one of the integrated unit IU1 and the integrated unit IU2 accommodated in the accommodation space SP. Therefore, the imaging unit 1 may include a heat radiating member that promotes heat radiation of at least one of the integrated unit IU1 and the integrated unit IU2. For example, in the accommodation space SP, heat may be generated from at least one of the face camera 11, the iris camera 12, the LED unit 13, the reflecting mirror 14, the drive motor 15, the display 16, the arithmetic apparatus 17, and the storage apparatus 18 that are accommodated in the accommodation space SP. Therefore, the imaging unit 1 may include a heat radiating member that promotes heat radiation of at least one of the face camera 11, the iris camera 12, the LED unit 13, the reflecting mirror 14, the drive motor 15, the display 16, the arithmetic apparatus 17, and the storage apparatus 18.

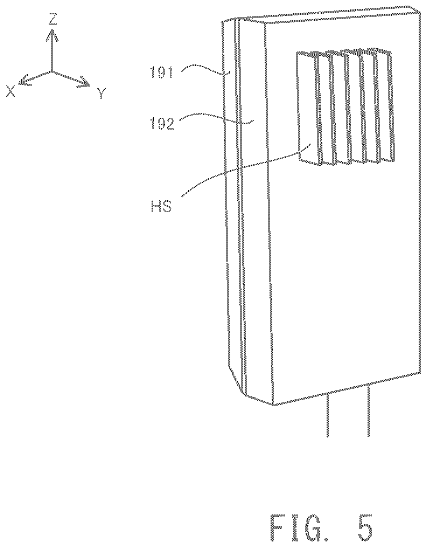

As an example, a part of the housing 19 may be used as the heat radiating member. In the example illustrated in FIG. 4, a vicinity part 196 located in the vicinity of the integrated unit IU1 of the housing 19 is used as a heat radiating member that promotes heat radiation of the integrated unit IU1. As another example, a different member from the housing 19 may be used as the heat radiating member. For example, as illustrated in FIG. 5, a heat sink HS mounted in the vicinity of the integrated unit IU2 of the housing 19 may be used as a heat radiating member that promotes heat radiation of the integrated unit IU2.

### (2-3) Arrangement Aspect of Face camera 11, Iris camera 12, The LED unit 13, Reflecting Mirror 14 and Drive motor 15

Next, with reference to FIG. 6 to FIG. 7, an example of an arrangement aspect of the face camera 11, the iris camera 12, the LED unit 13, the reflecting mirror 14, and the drive motor 15 in the accommodation space SP inside the housing 19, (i.e., a configuration of the integrated unit IU1) will be described. FIG. 6 is a perspective view illustrating an example of the arrangement aspect of the face camera 11, the iris camera 12, the LED unit 13, the reflecting mirror 14, and the drive motor 15. FIG. 7 is a perspective view illustrating an example of the arrangement aspect of the face camera 11, the iris camera 12, the LED unit 13, the reflecting mirror 14n and the drive motor 15, in a state of separating them from each other.

The arrangement aspects of the face camera 11, the iris camera 12, the LED unit 13, the reflecting mirror 14 and the drive motor 15 illustrated in FIG. 6 and FIG. 7 are merely an example. Therefore, the arrangement aspects of the face camera 11, the iris camera 12, the LED unit 13, the reflecting mirror 14, and the drive motor 15 are not limited to those illustrated in FIG. 6 and FIG. 7.

As illustrated in FIG. 6 and FIG. 7, the face camera 11, the iris camera 12, the LED unit 13, the reflecting mirror 14, and the drive motor 15 are mounted on the unit base 190. The unit base 190 includes a plate-shaped bottom member 1901 along an XY plane, and a pair of plate-shaped side wall members 1902 extending upward along a Z-axis direction from both ends of the bottom member 1901. An X-axis and a Y-axis in FIG. 6 and FIG. 7 may be horizontal axes perpendicular to each other. AZ axis in FIG. 6 and FIG. 7 may be a vertical axis perpendicular to the X-axis and the Y-axis.

The drive motor 15 is mounted on the bottom member 1901. A camera base 151 is mounted on a rotating shaft 150 of the drive motor 15. Therefore, the camera base 151 is rotatable by the drive motor 15 around the rotating shaft 150 of the drive motor 15. That is, the camera base 151 is rotatable around a predetermined rotation axis that defines a rotation center of the rotating shaft 150 of the drive motor 15. A rotation axis of the camera base 151 is an axis extending along a direction in which the rotating shaft 150 of the drive motor 15 extends. In the examples illustrated in FIG. 6 and FIG. 7, the rotating shaft of the drive motor 15 (i.e., the rotation axis of the camera base 151) is parallel to the X-axis. T face camera 11 and the iris camera 12 are mounted on the camera base 151. Specifically, the face camera 11 and the iris camera 12 are mounted on the camera base 151, such that the face camera 11 and the iris camera 12 are aligned along the rotation axis. Therefore, each of the face camera 11 and the iris camera 12 is rotatable by the drive motor 15 around the rotation axis of the camera base 151.

The face camera 11 is mounted on the camera base 151 such that an optical axis of the face camera 11 (e.g., an optical axis of an optical system such as a lens) intersects the rotation axis of the camera base 151. The iris camera 12 is mounted on the camera base 151 such that an optical axis of the iris camera 12 (e.g., an optical axis of an optical system such as a lens) intersects the rotation axis of the camera base 151. Therefore, the drive motor 15 is capable of rotating each of the face camera 11 and the iris camera 12 in the tilt direction. That is, the drive motor 15 is capable of rotating each of the face camera 11 and the iris camera 12 such that the imaging range of each of the face camera 11 and the iris camera 12 moves in a vertical direction. Therefore, even when the position of the face of the target person P changes in the vertical direction depending on the height of the target person P, the face camera 11 is capable of properly imaging the face of the target person P by moving the imaging range in the vertical direction. Similarly, even when the position of the eye of the target person P changes in the vertical direction depending on the height of the target person P, the iris camera 12 is capable of properly imaging the eye (especially, the iris) of the target person P by moving the imaging range in the vertical direction.

A camera cover 152 is further mounted on the camera base 151. The camera cover 152 is a member for partially covering the face camera 11 and the iris camera 12. Specifically, the camera cover 152 includes a cover member 1521 that partially covers the face camera 11 and a cover member 1522 that partially covers the iris camera 12. An opening 15211 that allows the light entering the face camera 11 from the target person P to pass through, may be formed in the cover member 1521. An opening 15221 that allows the return light entering the iris camera 12 from the target person P to pass through, may be formed in the cover member 1522. The camera cover 152 may further include a cover member 1523 for partially covering the drive motor 15.

The reflecting mirror 14 is mounted on the camera cover 152. Therefore, the reflecting mirror 14 is rotatable by the drive motor 15 around the rotation axis of the camera base 151. Specifically, each of both ends of the camera cover 152 functions as a mirror mounting member 1524 that is mountable by the reflecting mirror 14. Therefore, in the second example embodiment, the imaging unit 1 includes a pair of reflecting mirrors 14 that are respectively mounted on the both ends of the camera cover 152. The reflecting mirror 14 is mounted on the camera cover 152 (especially, the mirror mounting member 1524) such that the reflecting mirror 14 intersects the rotation axis of the camera base 151. That is, the reflecting mirror 14 is mounted on the camera cover 152 (especially, the mirror mounting member 1524) such that the rotation axis of the camera base 151 is an axis capable of passing through the reflecting mirror 14.

On the other hand, the LED unit 13 is mounted on the side wall member 1902. That is, the LED unit 13 is mounted on a different member from the bottom member 1901 on which the face camera 11, the iris camera 12, the reflecting mirror 14, and the drive motor 15 are mounted. The LED unit 13 is disposed at a different position from positions where the face camera 11, the iris camera 12, the reflecting mirror 14, and the drive motor 15 are disposed. The LED unit 13 is disposed at a position away from the positions where the face camera 11, the iris camera 12, the reflecting mirror 14 and the drive motor 15 are disposed. Therefore, the drive motor 15 may not rotate the LED unit 13. The LED unit 13 may be fixed to the side wall member 1902. The LED unit 13 may not be movable.

Specifically, a pair of LED units 13 is mounted on the pair of side wall members 1902, respectively. As illustrated in FIG. 6 and FIG. 7, the camera base 151 and the camera cover 152 are disposed between the pair of side wall members 1902. The pair of LED units 13 is respectively mounted on the pair of side wall members 1902 such that the pair of LED units 13 respectively emits the illumination light toward the pair of reflecting mirrors 14 mounted respectively on the both ends of the camera cover 152. That is, the pair of LED units 13 is arranged to face in a different direction from a direction of the target person P, instead of facing in the direction of the target person P. The pair of LED units 13 is arranged to emit the illumination light in a different direction from the direction of the target person P, instead of directly emitting the illumination light in the direction of the target person P. Consequently, there is a low possibility that the LED unit 13 is visually recognized from the outside of the imaging unit 1, and the design of the imaging unit 1 is thus improved.

The reflecting mirror 14 is mounted on the camera cover 152 such that the reflecting mirror 14 is capable of reflecting the illumination light emitted from the LED unit 13 toward the target person P. In this case, when the drive motor 15 rotates the reflecting mirror 14, the irradiation position of the illumination light emitted from the LED unit 13 toward the target person P through the reflecting mirror 14 moves in the vertical direction. Therefore, even when the position of the eye of the target person P changes in the vertical direction depending on the height of the target person P, the LED unit 13 is capable of properly illuminating the eye (especially, the iris) of the target person P with the illumination light by moving the irradiation position of the illumination light in the vertical direction with the reflecting mirror 14.

The iris camera 12, the reflecting mirror 14, and the LED unit 13 are preferably aligned in advance such that the illumination light emitted from the LED unit 13 is irradiated to the iris of the target person P through the reflecting mirror 14 and such that the return light from the iris of the target person P enters the iris camera 12, even when the drive motor 15 rotates the iris camera 12 and the reflecting mirror 14. In other words, even when the drive motor 15 rotates the iris camera 12 and the reflecting mirror 14, a positional relation among the iris camera 12, the reflecting mirror 14, and the LED unit 13 is preferably maintained in an ideal positional relation in which the illumination light emitted from the LED unit 13 is irradiated to the iris of the target person P through the reflecting mirror 14 and in which the return light from the iris of the target person P enters the iris camera 12.

The LED unit 13 may be mounted on the side wall member 1902 such that an optical axis of the LED unit 13 is coaxial with the rotating shaft 150 of the drive motor 15. The LED unit 13 may be mounted on the side wall member 1902 such that the optical axis of the LED unit 13 is coaxial with the rotation axis of the camera base 151. The "optical axis of the LED unit 13" in the second example embodiment may mean a central axis of a beam of light when the illumination light emitted from the LED unit 13 is regarded as a single beam of light. The "optical axis of the LED unit 13" in the second example embodiment may mean an axis extending along a principal ray of the beam of light when the illumination light emitted from the LED unit 13 is regarded as a single beam of light.

### (2-4) Configuring of Authentication Server 2

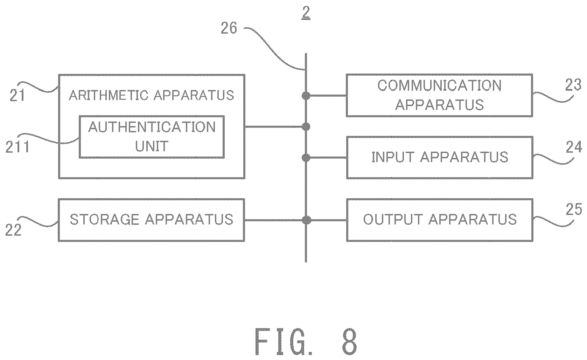

Next, with reference to FIG. 8, a configuration of the authentication server 2 will be described. FIG. 8 is a block diagram illustrating the configuration of the authentication server 2.

As illustrated in FIG. 8, the authentication server 2 includes an arithmetic apparatus 21, a storage apparatus 22, and a communication apparatus 23. Furthermore, the authentication server 2 may include an input apparatus 24 and an output apparatus 25. The authentication server 2, however, may not include at least one of the input apparatus 24 and the output apparatus 25. The arithmetic apparatus 21, the storage apparatus 22, the communication apparatus 23, the input apparatus 24, and the output apparatus 25 may be connected through a data bus 26.

The arithmetic apparatus 21 includes at least one of a CPU (Central Processing Unit), a GPU (Graphics Processing Unit), a FPGA (Field Programmable Gate Array), a DSP (Demand-Side Platform), and an ASIC (Application Specific Integrated Circuit), for example. The arithmetic apparatus 21 reads a computer program. For example, the arithmetic apparatus 21 may read a computer program stored in the storage apparatus 22. For example, the arithmetic apparatus 21 may read a computer program stored by a computer-readable and non-transitory recording medium, by using a not-illustrated recording medium reading apparatus provided in the authentication server 2. The arithmetic apparatus 21 may acquire (i.e., download or read) a computer program from a not-illustrated apparatus disposed outside the authentication server 2, through the communication apparatus 23 (or another communication apparatus). The arithmetic apparatus 21 executes the read computer program. Consequently, a logical functional block for performing an operation to be performed by the authentication server 2 (e.g., the authentication operations described above) is realized or implemented in the arithmetic apparatus 21. That is, the arithmetic apparatus 21 is allowed to function as a controller for realizing or implementing the logical functional block for performing an operation (in other words, processing) to be performed by the authentication server 2.

FIG. 8 illustrates an example of the logical functional block realized or implemented in the arithmetic apparatus 21 to perform the authentication operation. As illustrated in FIG. 8, an authentication unit 211 is realized or implemented in the arithmetic apparatus 21. The authentication unit 211 is a specific example of the "authentication unit" described in Supplementary Note later. The authentication unit 211 acquires the iris image IMG_I from the imaging unit 1 and authenticates the target person P based on the acquired iris image IMG_I.

The storage apparatus 22 is configured to store desired data. For example, the storage apparatus 22 may temporarily store a computer program to be executed by the arithmetic apparatus 21. The storage apparatus 22 may temporarily store data that are temporarily used by the arithmetic apparatus 21 when the arithmetic apparatus 21 executes the computer program. The storage apparatus 22 may store data that are stored by the authentication server 2 for a long time. The storage apparatus 22 may include at least one of a RAM (Random Access Memory), a ROM (Read Only Memory), a hard disk apparatus, a magneto-optical disk apparatus, a SSD (Solid State Drive), and a disk array apparatus. That is, the storage apparatus 22 may include a non-transitory recording medium.

The communication apparatus 23 is configured to communicate with the imaging unit 1 through a not-illustrated communication network. In the second example embodiment, the communication apparatus 23 receives (i.e., acquires) the person image IMG (especially, the iris image IMG_I) from the imaging unit 1.

The input apparatus 24 is an apparatus that receives an input of information to the authentication server 2 from an outside of the authentication server 2. For example, the input apparatus 24 may include an operating apparatus (e.g., at least one of a keyboard, a mouse, and a touch panel) that is operable by an operator of the authentication server 2. For example, the input apparatus 24 may include a reading apparatus that is configured to read information recorded as data on a recording medium that can be externally attached to the authentication server 2.

The output apparatus 25 is an apparatus that outputs information to the outside of the authentication server 2. For example, the output apparatus 25 may output information as an image. That is, the output apparatus 25 may include a display apparatus (a so-called display) that is configured to display an image indicating the information that is desirably outputted. For example, the output apparatus 25 may output information as audio/sound. That is, the output apparatus 25 may include an audio apparatus (a so-called speaker) that is configured to output the audio/sound. For example, the output apparatus 25 may output information onto a paper surface. That is, the output apparatus 25 may include a print apparatus (a so-called printer) that is configured to print desired information on the paper surface.

### (2-5) Imaging Operation of Imaging Unit 1 and Authentication Operation of Authentication Server 2

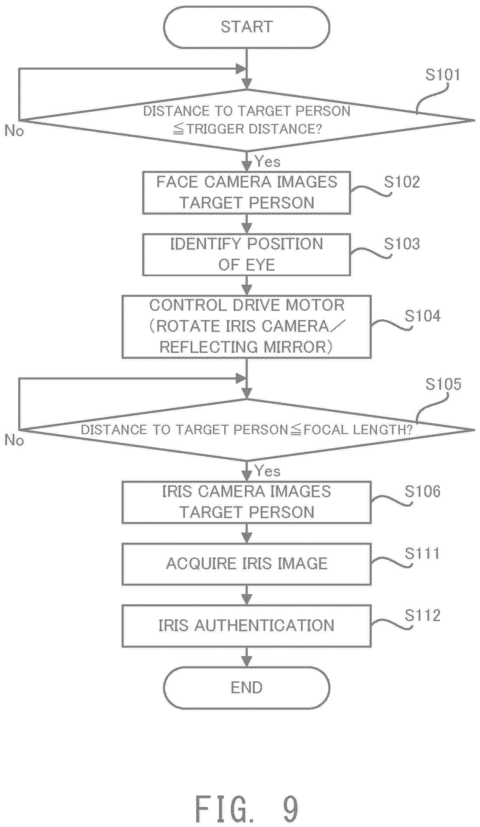

Next, with reference to FIG. 9, the imaging operation performed by the imaging unit 1 and the authentication operation performed by authentication server 2 will be described. FIG. 9 is a flowchart illustrating a flow of the imaging operation performed by the imaging unit 1 and the authentication operation performed by the authentication server 2.

As illustrated in FIG. 9, first, the imaging unit 1 performs the imaging operation (step S101 to step S106). The authentication server 2 then performs the authentication operation (step S111 to step S 112).

Specifically, the imaging control unit 171 of the imaging unit 1 determines whether or not a distance from the imaging unit 1 to the target person P is less than or equal to a predetermined trigger distance (step S101). For example, the imaging control unit 171 may determine whether the distance from the imaging unit 1 to the target person P is less than or equal to the trigger distance based on a detection result of a not-illustrated distance sensor that is configured to detect the distance from the imaging unit 1 to the target person P. The trigger distance may be a distance from the imaging unit 1 to a position where the face camera 11 is in focus (i.e., a focal plane of the face camera 11). In this case, the operation of determining whether or not the distance from the imaging unit 1 to the target person P is less than or equal to the trigger distance, is equivalent to an operation of determining whether or not the target person P is located at the position where the face camera 11 is in focus.

As a result of the determination in the step S101, when it is determined that the distance from the imaging unit 1 to the target person P is not less than or equal to the trigger distance (the step S101: No), the imaging control unit 171 continues to determine whether or not the distance from the imaging unit 1 to the target person P is less than or equal to the trigger distance.

On the other hand, as a result of the determination in the step S101, when it is determined that the distance from the imaging unit 1 to the target person P is less than or equal to the trigger distance (the step S101: Yes), the imaging control unit 171 controls the face camera 11 to image the face of the target person P (step S102). As a consequence, the face camera 11 images the face of the target person P (step S102).

Thereafter, the rotation control unit 172 acquires the face image IMG_F from the face camera 11 and identifies the position of the eye of the target person P (especially, the position in the vertical direction, and the position in the Z-axis direction, for example) based on the acquired face image IMG_F (step S103). Thereafter, the rotation control unit 172 controls the drive motor 15 to rotate the iris camera 12 and the reflecting mirror 14 such that the reflecting mirror 14 is capable of reflecting the illumination light emitted from the LED unit 13 toward the eye located at the position identified in the step S103 and such that the iris camera 12 is capable of receiving the return light from the eye located at the position identified in the step S103 (step S104). Consequently, the iris camera 12 is in a state capable of imaging the eye of the target person P illuminated with the illumination light.

Thereafter, the imaging control unit 171 determines whether or not the distance from the imaging unit 1 to the target person P is less than or equal to a predetermined focal length (step S105). For example, the imaging control unit 171 may determine whether or not the distance from the imaging unit 1 to the target person P is less than or equal to the focal length based on a detection result of a not-illustrated distance sensor that is configured to detect the distance from the imaging unit 1 to the target person P. The focal length may be a distance from the imaging unit 1 to a position where the iris camera 12 is in focus (i.e., a focal plane of the iris camera 12). In this instance, the operation of determining whether or not the distance from the imaging unit 1 to the target person P is less than or equal to the focal length, is equivalent to an operation of determining whether or not the target person P is located at the position where the iris camera 12 is in focus. Note that the focal length is typically less than the trigger distance described above.

As a result of the determination in the step S105, when it is determined that the distance from the imaging unit 1 to the target person P is not less than or equal to the focal length (the step S105: No), the imaging control unit 171 continues to determine whether or not the distance from the imaging unit 1 to the target person P is less than or equal to the focal length.

On the other hand, as a result of the determination in the step S105, when it is determined that the distance from the imaging unit 1 to the target person P is less than or equal to the focal length (the step S105: Yes), the imaging control unit 171 controls the iris camera 12 to image the eye (especially, the iris) of the target person P (step S106). Consequently, the iris camera 12 images the eye of the target person P (step S106).

When the iris camera 12 images the eye of the target person P, the authentication server 2 starts the authentication operation (step S111 to step S112). Specifically, the authentication unit 211 of the authentication server 2 acquires the iris image IMG_I from the iris camera 12 through the communication apparatus 23 (step S111). The authentication unit 211 then authenticates the target person P based on the iris image IMG_I acquired in the step S111 (step S112).

As a result of the authentication in the step S112, when the authentication of the target person P is successful, the authentication unit 211 may perform an operation to be performed when the authentication of the target person P is successful. For example, as described above, when the information processing system SYS2 is used to manage/control the entry of the target person P to the entry restricted area, the authentication unit 211 may set the state of the gate apparatus disposed at the entrance/exit of the entry restricted area into the opening state in which the gate apparatus does not block the passage of the target person P. For example, as described above, when the information processing SYS2 is used to manage/control a payment for goods or services by the target person P, the authentication unit 211 may complete the payment by a settlement method associated with the target person P.

On the other hand, as a result of the authentication in the step S112, when the authentication of the target person P is not successful, the authentication unit 211 may perform an operation to be performed when the authentication of the target person P is not successful. For example, as described above, when the information processing system SYS2 is used to manage/control the entry of the target person P to the entry restricted area, the authentication unit 211 may set the state of the gate apparatus disposed at the entrance/exit of the entry restricted area into the closing state in which the gate apparatus prevents the passage of the target person P. For example, as described above, when the information processing SYS2 is used to manage/control a payment for goods or services by the target person P, the authentication unit 211 may not complete the payment by the settlement method associated with the target person P.

### (2-6) Technical Effect of Information processing System SYS2

As described above, according to the information processing system SYS2 in the second example embodiment, the drive motor 15 may rotate each of the face camera 11, the iris camera 12, and the reflecting mirror 14, but may not rotate the LED unit 13. Consequently, in the second example embodiment, as compared with a second comparative example in which the LED unit 13 is rotated with each of the face camera 11, the iris camera 12, and the reflecting mirror 14, wires connected to the LED unit 13 are less likely to be disconnected due to the rotation of each of the face camera 11, the iris camera 12, and the reflecting mirror 14. Specifically, in the second example embodiment, as compared with the second comparative example, there is a low possibility that a bending stress caused by the rotation of each of the face camera 11, the iris camera 12, and the reflecting mirror 14 is applied to the wires connected to the LED unit 13. Consequently, in the second example embodiment, as compared with the second comparative example, the wires connected to the LED unit 13 are less likely to be disconnected by the bending stress caused by the rotation of each of the face camera 11, the iris camera 12, and the reflecting mirror 14. Therefore, the imaging unit 1 in the second example embodiment is capable of solving the first technical problem that the wires connected to the LED unit 13 may be disconnected.

In addition, when the LED unit 13 does not rotate, it is possible to shorten the wires connected to the LED unit 13 than those when the LED unit 13 rotates. Consequently, in the second example embodiment, it is possible to reduce the weight of the wires connected to the LED unit 13. That is, it is possible to reduce the weight of the imaging unit 1.

In addition, in the information processing system SYS2 in the second example embodiment, The LED unit 13 may not be rotationally moved with each of the face camera 11, the iris camera 12, and the reflecting mirror 14 that are rotationally moved by the drive motor 15. Therefore, in the second example embodiment, as compared with the second comparative example in which the LED unit 13 is rotationally moved with each of the face camera 11, the iris camera 12, and the reflecting mirror 14, the LED unit 13 may be disposed at a position away from each of the face camera 11, the iris camera 12, the reflecting mirror 14, and the drive motor 15. That is, in the second example embodiment, as compared with the second comparative example, the LED unit 13 may be separated relatively significantly from each of the face camera 11, the iris camera 12, the reflecting mirror 14, and the drive motor 15. In other words, the LED unit 13 may be disposed at a different position from positions where the face camera 11, the iris camera 12, the reflecting mirror 14, and the drive motor 15 are disposed. Specifically, the LED unit 13 is mounted on the side wall member 1902 that is different from the bottom member 1901 on which each of the face camera 11, the iris camera 12, and the reflecting mirror 14 is mounted. Consequently, in the second example embodiment, as compared with the second comparative example, a heat generated in the LED unit 13 due to the emission of the illumination light is hardly transmitted to each of the face camera 11, the iris camera 12, and the reflecting mirror 14. Therefore, in the second example embodiment, as compared with the second comparative example, an influence on each of the face camera 11, the iris camera 12 and the reflecting mirror 14, which is received from the heat of the LED unit 13, is reduced. Therefore, in the second example embodiment, as compared with the second comparative example, each of the face camera 11, the iris camera 12, and the reflecting mirror 14 is likely to operate normally, without being influenced by the heat caused by the emission of the illumination light in the LED unit 13. Therefore, the imaging unit 1 in the second example embodiment is capable of solving the second technical problem that each of the face camera 11, the iris camera 12 and the reflecting mirror 14 may not operate normally due to the influence of the heat of the LED unit 13.

In addition, in the information processing system SYS2 in the second example embodiment, the LED unit 13 irradiates the target person P with the illumination light through the reflecting mirror 14. Therefore, the LED unit 13 may not directly emit the illumination light toward the target person P. Consequently, there is a low possibility that the LED unit 13 is visually recognized from the outside of the imaging unit 1. Therefore, the design of the imaging unit 1 is improved.

In addition, in the information processing system SYS2 in the second example embodiment, since the LED unit 13 may not be rotated, the LED unit 13 can be disposed at a fixed position where it is not driven by the drive motor 15. Therefore, in the second example embodiment, as compared with the second comparative example in which the LED unit 13 is rotated with each of the face camera 11, the iris camera 12, and the reflecting mirror 14, a positional relation between the LED unit 13 and each of the face camera 11, the iris camera 12, and the reflecting mirror 14, is less likely to deviate from the ideal positional relation. As described above, in the ideal positional relation, a positional relation between the LED unit 13 and each of the iris camera 12 and the reflecting mirror 14 may mean a positional relation in which the illumination light emitted from the LED unit 13 is irradiated to the iris of the target person P through the reflecting mirror 14 and in which the return light from the iris of the target person P enters the iris camera 12, even when the drive motor 15 rotates the iris camera 12 and the reflecting mirror 14. Specifically, the information processing apparatus described in Patent Literature 1 described above separately includes a motor for moving a camera that images a target t and a motor for moving an illuminator for illuminating the target. Therefore, in the information processing apparatus described in Patent Literature 1, in order to maintain a state where a positional relation between the camera and the illuminator is an ideal positional relation, it is necessary to control the two motors such that the motor for moving the camera and the motor for moving the illuminator operate in synchronization with each other with high accuracy. Consequently, the information processing apparatus described in Patent Literature 1 has a third technical problem that the positional relation between the camera and the illuminator is likely to deviate from the ideal positional relation, as compared with a case where the illuminator is not moved while the camera is moved, or where the camera is not moved while the illuminator is moved. In the imaging unit 1 in the second example embodiment, however, since the LED unit 13 may not be rotated, there is a low possibility that the third technical problem in Patent Literature 1 occurs. That is, the imaging unit 1 in the second example embodiment is capable of reducing the possibility that the positional relation between the LED unit 13 and each of the face camera 11, the iris camera 12, and the reflecting mirror 14 deviates from the ideal positional relation. Therefore, the imaging unit 1 in the second example embodiment is capable of solving the third technical problem that the positional relation between the LED unit 13 and each of the face camera 11, the iris camera 12, and the reflecting mirror 14 may deviate from the ideal positional relation.

Furthermore, in the second example embodiment, the imaging unit 1 may include a heat radiating member capable of radiating a heat of the accommodation space SP inside the housing 19 to the outside of the housing 19. Therefore, as compared with a case where the imaging unit 1 does not include the heat radiating member, the imaging unit 1 is less likely to be influenced by the heat generated in the accommodation space SP (e.g., heat generated from an apparatus accommodated in the accommodation space SP). Therefore, the imaging unit 1 is capable of properly performing the imaging operation, without being influenced by the heat generated in the accommodation space SP.

In the second example embodiment, the imaging unit 1 identifies the position of the eye of the target person P based on the face image IMG_F, and rotates the iris camera 12 and the reflecting mirror 14 such that the reflecting mirror 14 is capable of reflecting the illumination light emitted from the LED unit 13 toward the eye located at the identified position and such that the iris camera 12 is capable of receiving the return light from the eye located at the identified position. Therefore, the imaging unit 1 is capable of properly illuminating the eye of the target person P with the illumination light and properly imaging the eye of the target person P, even when the position of the eye of the target person P changes in the vertical direction depending on the height of the target person P.

In the second example embodiment, the imaging unit 1 is capable of rotating the face camera 11, the iris camera 12, and the reflecting mirror 14. Therefore, the imaging unit 1 is capable of moving the imaging range of the face camera 11 in the vertical direction, moving the imaging range of the iris camera 12 in the vertical direction, and moving the illumination position of the illumination light reflected by the reflecting mirror 14 in the vertical direction. Therefore, the imaging unit 1 is capable of properly imaging the face of the target person P and the face of the target person P, and the eye of the target person P, properly image the eye of the target person P, and properly illuminating the eye of the target person P with the illumination light, even when the positions of the face and the eye change in the vertical direction depending on the height of the person P.

Furthermore, in the second example embodiment, the LED unit 13 may be mounted on the side wall member 1902 such that the optical axis of the LED unit 13 is coaxial with the rotating shaft 150 of the drive motor 15 (i.e., the rotation axis of the camera base 151). Therefore, even when the drive motor 15 rotates the reflecting mirror 14, the illumination light emitted from the LED unit 13 enters the reflecting mirror 14. Therefore, the illumination light can be emitted toward the target person P from the reflecting mirror 14, even when the drive motor 15 rotates the reflecting mirror 14. In this case, although an emission angle of the illumination light emitted from the reflecting mirror 14 is changed with the rotation of the reflecting mirror 14, an emission position of the illumination light emitted from the reflecting mirror 14 is not changed. Consequently, even when the drive motor 15 rotates the iris camera 12 and the reflecting mirror 14, the illumination light emitted from the LED unit 13 is likely to be irradiated to the iris of the target person P through the reflecting mirror 14. That is, even when the drive motor 15 rotates the iris camera 12 and the reflecting mirror 14, the ideal positional relation among the iris camera 12, the reflecting mirror 14, and The LED unit 13 described above is easily maintained.

### (2-7) Modified Examples

### (2-7-1) First Modified Example

In the above description, the authentication unit 211 of the authentication server 2 starts the authentication operation when it is determined that the distance from the imaging unit 1 to the target person P is less than or equal to the focal length. The authentication unit 211, however, may further determine whether or not the target person P agrees that the authentication server 2 authenticates the target person P before starting the authentication operation, when it is determined that the distance from the imaging unit 1 to the target person P is less than or equal to the focal length. That is, the authentication unit 211 may further determine whether or not the target person P has an intention to allow the authentication server 2 to authenticate the target person P. When it is determined that the target person P has an intention to allow the authentication server 2 to authenticate the target person P, the authentication unit 211 may start the authentication operation. On the other hand, when it is not determined t that the target person P has an intention to allow the authentication server 2 to authenticate the target person P, the authentication unit 211 may not start the authentication operation.

As an example, the authentication unit 211 may determine whether or not the target person P makes a predetermined gesture indicating that the target person P has an intention to allow the authentication server 2 to authenticate the target person P, based on the face image IMG_F. An example of the predetermined gestures is a gesture in which the target person P is looking toward the imaging unit 1. In this instance, the authentication unit 211 may estimate the line of sight of the target person P based on face image IMG_F by using an existing line-of-sight estimation method, thereby determining whether or not the target person P is looking toward the imaging unit 1.

As another example, when the iris image IMG_I is acquired, the authentication unit 211 may determine that the target person P has an intention to allow the authentication server 2 to authenticate the target person P. This is because, when the iris image IMG_I is acquired, it is assumed that the target person P is looking at the iris camera 12 and the target person P thus has an intention to allow the authentication server 2 to authenticate the target person P. In addition, when the iris image IMG_I is not acquired, the authentication unit 211 may determine whether or not the target person P has an intention to allow the authentication server 2 to authenticate the target person P, by using another method. For example, the authentication unit 211 may determine whether or not the target person P has an intention to allow the authentication server 2 to authenticate the target person P, based on operation details of the target person P on the display 16 that is allowed function as a touch panel.

The authentication unit 211 may control the display 16 of the imaging unit 1 to display a UI (User Interface) screen for asking the target person P whether or not the target person P has an intention to allow the authentication server 2 to authenticate the target person P. For example, the authentication unit 211 may control the display 16 to display a UI (User Interface) screen for encouraging the target person P to make a predetermined gesture indicating that the target person P has an intention to allow the authentication server 2 to authenticate the target person P.

The authentication unit 211 may determine whether or not the target person P has an intention to allow the authentication server 2 to authenticate the target person P, in at least a part of a period when the rotation control unit 172 controls the drive motor 15 to rotate the iris camera 12 and the reflecting mirror 14. A certain time is required in order that the rotation control unit 172 controls the drive motor 15 to rotate the iris camera 12 and the reflecting mirror 14, and the authentication unit 211 may determine whether or not the target person P has an intention to allow the authentication server 2 to authenticate the target person P at this time so as to effectively utilize this time.

### (2-7-2) Second Modified Example

When the authentication of the target person P is successful, the authentication unit 211 may transmit to the imaging unit 1 a control signal for controlling the display 16 to display the UI screen for notifying the target person P that the authentication is successful. As a consequence, the target person P can understand that the authentication is successful, by confirming the UI screen displayed on the display 16. In this instance, the authentication unit 211 may display the UI screen for notifying the target person P that the authentication is successful, by using a display method that protects personal information about the target person P. For example, the authentication unit 211 may display a UI screen including an avatar set by the target person, as the UI screen for notifying the target person P that the authentication is successful.

### (2-7-3) Third Modified Example

When the face camera 11 images the face of the target person P, the imaging control unit 171 may control the display 16 to display a predetermined UI screen. The predetermined UI screen may include, for example, a screen for encouraging the target person P to turn the face to the face camera 11. The predetermined UI screen may include, for example, a screen for encouraging the target person P to stand in front of the face camera 11.

Even when the iris camera 12 images the eye of the target person P, as in the face camera 11 images the face of the target person P, the imaging control unit 171 may control the display 16 to display a predetermined UI screen. The predetermined UI screen may include, for example, a screen for encouraging the target person P to turn the face to the iris camera 12. The predetermined UI screen may include, for example, a screen for encouraging the target person P to stand in front of the iris camera 12. The predetermined UI screen may include, for example, a screen for encouraging the target person P to open the eye wide. The predetermined UI screen may include an animation UI including a first picture for opening the eye of the target person P wide and a second picture, displayed next to the first picture, for making a pupil of the target person P small (e.g., a picture imitating a flashlight using visible light).

### (2-7-4) Fourth Modified Example

When a payment is completed based on the result of the authentication operation, the authentication unit 211 may further determine whether or not the target person P has an intention to complete the payment. As an example, before completing the payment, the authentication unit 211 may acquire the face image IMG F from the face camera 11 and may determine whether or not the target person P makes a predetermined gesture indicating that the target person P has an intention to complete the payment, based on the face image IMG_F. An example of the predetermined gesture is at least one of an eye blink and a nod. At this time, the authentication unit 211 may control the display 16 to display an UI screen for asking the target person P whether or not to have an intention to complete the payment. When it is determined that the target person P has an intention to complete the payment, the authentication unit 211 may complete the payment. When it is not determined that the target person P has an intention to complete the payment, the authentication unit 211 may not complete the payment.

### (2-7-5) Other Modified Examples

In the above description, the pair of reflecting mirrors 14 is disposed inside the pair of LED units 13. The pair of LED units 13, however, may be disposed inside the pair of reflecting mirrors 14. That is, an arrangement position of the pair of LED units 13 and an arrangement position of the pair of reflecting mirrors 14 may be inverted.

In the above description, the authentication server 2 authenticates the target person P by using the iris image IMG_I. The authentication server 2, however, may authenticate the target person P by using the iris image IMG I and may authenticate the target person P by using the face image IMG_F. That is, the authentication server 2 may function as an authentication apparatus having a multi-modal function. Alternatively, the authentication server 2 may authenticate the target person P by using face image IMG_F, without authenticating the target person P by using the iris image IMG_I.

In the above description, the imaging unit 1 and the authentication server 2 are separate apparatuses. The imaging unit 1 and the authentication server 2, however, may be integrated. For example, the arithmetic apparatus 17 of the imaging unit 1 may include the authentication unit 211 of the authentication server 2.

The arithmetic apparatus 21 of the authentication server 2 may include at least a part of processing blocks of the arithmetic apparatus 17 of the imaging unit 1. For example, the arithmetic apparatus 21 of the authentication server 2 may include at least one of the imaging control unit 171, the rotation control unit 172, and the display control unit 173 provided in the arithmetic apparatus 17 of the imaging unit 1.

The arithmetic apparatus 17 of the imaging unit 1 may include at least a part of processing blocks of the arithmetic apparatus 21 of the authentication server 2. For example, the arithmetic apparatus 17 of the imaging unit 1 may include the authentication unit 211 provided in the arithmetic apparatus 21 of the authentication server 2.

### (3) Third Example Embodiment

Next, an information processing system and an information processing apparatus in a third example embodiment will be described. The following describes an information processing system SYS3 to which the information processing system and the information processing apparatus in the third example embodiment are applied. The information processing system SYS3 in the third example embodiment is different from the information processing system SYS2 in the second example embodiment, in that it includes an imaging unit 1c in place of the imaging unit 1. Other features of the information processing system SYS3 may be the same as those of the information processing system SYS2.

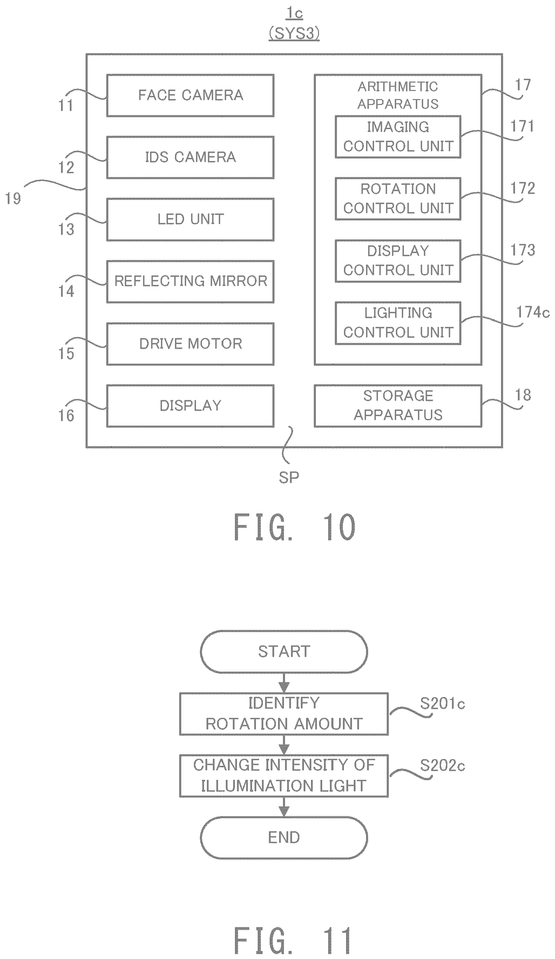

Hereinafter, with reference to FIG. 10, the imaging unit 1c in the third example embodiment will be described. FIG. 10 is a block diagram illustrating a configuration of the imaging unit 1c in the third example embodiment. In the following, a detailed description of the previously described components will be omitted by giving the same reference numerals.

As illustrated in FIG. 10, the imaging unit 1c in the third example embodiment is different from the imaging unit 1 in the second example embodiment, in that the arithmetic apparatus 17 includes a lighting control unit 174c. The lighting control unit 174c is a specific example of the "intensity control unit" described in Supplementary Note later. Other features of the imaging unit 1c may be the same as those of the imaging unit 1.

The lighting control unit 174c changes intensity of the illumination light emitted from the LED unit 13, based on a rotation amount (i.e., a driving amount) of the camera base 151 by the drive motor 15. As described above, since the face camera 11, the iris camera 12 and the reflecting mirror 14 rotate with the rotation of the camera base 151, the rotation amount of the camera base 151 may be regarded as equivalent to a rotation amount of each of the face camera 11, the iris camera 12 and the reflecting mirror 14. In this instance, it can be said that lighting control unit 174c changes the intensity of the illumination light emitted from the LED unit 13 based on the rotation amount of each of the face camera 11, the iris camera 12, and the reflecting mirror 14 by the drive motor 15.

The lighting control unit 174c may change the intensity of the illumination light in accordance with the flowchart illustrated in FIG. 11, when the drive motor 15 rotates the camera base 151 in the step S105 in FIG. 9 described above. Specifically, first, the lighting control unit 174c identifies the rotation amount of the camera base 151 by the drive motor 15 (step S201c). Since the rotation control unit 172 controls the camera base 151 by the drive motor 15, the lighting control unit 174c may identify the rotation amount, by acquiring information about the rotation amount of the camera base 151 from the rotation control unit 172. Thereafter, the lighting control unit 174c changes the intensity of the illumination light based on the rotation amount identified in the step S201c (step S202c).

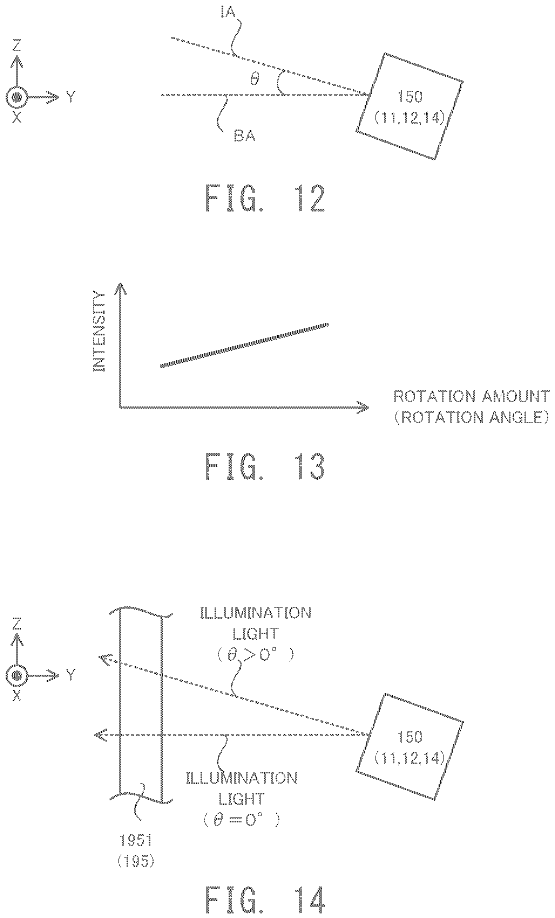

An example of a parameter indicating the rotation amount of the camera base 151 is a rotation angle θ of the camera base 151. The rotation angle θ of the camera base 151 may be an angle formed by a reference axis BA (e.g., the Y-axis that is a horizontal axis, and an axis perpendicular to the X-axis that is the rotation axis of the camera base 151) and an imaging axis IA extending along a direction in which the camera base 151 is directed, as illustrated in FIG. 12. As the imaging axis IA, the optical axis of the face camera 11 (e.g., the optical axis of the optical system of the face camera 11 (lens, etc.)) may be used. In this case, the rotation angle θ of the camera base 151 may mean a rotation angle of the face camera 11. As the imaging axis IA, the optical axis of the iris camera 12 (e.g., the optical axis of the optical system of the iris camera 12 (lens, etc.)) may be used. In this case, the rotation angle θ of the camera base 151 may mean a rotation angle of the iris camera 12. As the imaging axis IA, an axis extending along a traveling direction of the illumination light reflected by the reflecting mirror 14 may be used. In this case, the rotation angle θ of the camera base 151 may mean a rotation angle of the reflecting mirror 14.

The lighting control unit 174c may change the intensity of the illumination light such that, as the rotation angle θ is increased (i.e., the rotation amount is increased), the intensity of the illumination light is increased. The lighting control unit 174c may change the intensity of the illumination light such that the intensity of the illumination light when the rotation angle θ is a first angle, is higher than the intensity of the illumination light when the rotation angle θ is a second angle that is smaller than the first angle. Alternatively, the lighting control unit 174c may change the intensity of the illumination light such that, as the rotation angle θ is increased, the intensity of the illumination light is reduced. The lighting control unit 174c may change the intensity of the illumination light such that the intensity of the illumination light when the rotation angle θ is the first angle, is lower than the intensity of the illumination light when the rotation angle θ is the second angle that is smaller than the first angle.

As an example, as described above, when the opening 195 of the housing 19 through which the illumination light reflected by the reflecting mirror 14 passes, is filled with the optical member 1951 that allows near infrared light to pass through and that absorbs or reflects a part of visible light, the lighting control unit 174c may change the intensity of the illumination light such that, as the rotation angle θ is increased, the intensity of the illumination light is increased, as illustrated in FIG. 13. The reason is described below.

First, as illustrated in FIG. 14, as the rotation angle θ is increased, an incidence angle of the illumination light reflected by the reflecting mirror 14, entering the optical member 1951, is increased. As a result, as the rotation angle θ is increased, an optical path of the illumination light in the optical member 1951 becomes longer. As the optical path of the illumination light in the optical member 1951 becomes longer, an attenuation amount of the intensity of the illumination light by the optical member 1951 is increased. Consequently, as the rotation angle θ is increased, the intensity of the illumination light reaching the target person P is reduced. The same applies in the return light from the target person P, and as the rotation angle θ is increased, the intensity of the return light reaching the iris camera 12 is reduced. Therefore, if the intensity of the illumination light is fixed to be constant (i.e., not changed), when the rotation angle θ is changed, the imaging unit 1 may not be capable of illuminating the target person P with the illumination light of appropriate intensity, which is technically problematic. Furthermore, if the intensity of the illumination light is fixed to be constant (i.e., not changed), when the rotation angle θ is changed, the imaging unit 1 may not be capable of properly imaging the eye of the target person P, which is technically problematic.

In order to solve such technical problems, the lighting control unit 174c may change the intensity of the illumination light such that, as the rotation angle θ is increased, the intensity of the illumination light is increased. In this instance, even if the rotation angle θ is changed, the intensity of the illumination light reaching the target person P is not excessively reduced. Therefore, the imaging unit 1 is capable of illuminating the target person P with the illumination light of appropriate intensity. Furthermore, even if the rotation angle θ is changed, the intensity of the return light reaching the iris camera 12 is not excessively reduced. Therefore, the imaging unit 1 is capable of properly imaging the eye of the target person P.

### (4) Fourth Example Embodiment

Next, an information processing system and an information processing apparatus in a fourth example embodiment will be described. The following describes an information processing system SYS4 to which the information processing system and the information processing apparatus in the fourth example embodiment are applied. The information processing system SYS4 in the fourth example embodiment is different from the information processing system SYS2 in the second example embodiment in that it includes an imaging unit 1d in place of the imaging unit 1. Other features of the information processing system SYS4 may be the same as those of the information processing system SYS2.

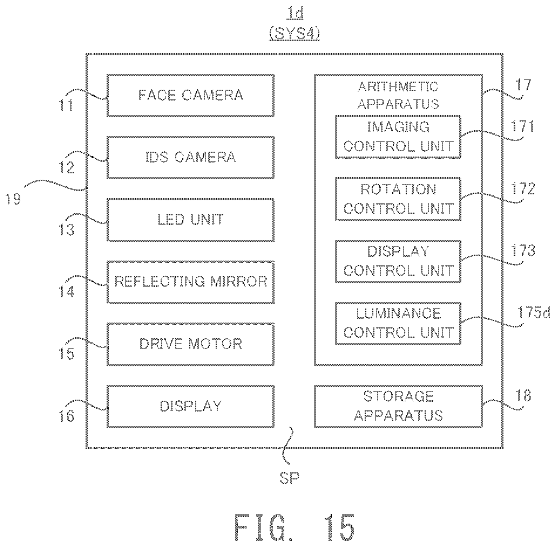

Hereinafter, with reference to FIG. 15, the imaging unit 1d in the fourth example embodiment will be described. FIG. 15 is a block diagram illustrating a configuration of the imaging unit 1d in the fourth example embodiment.

As illustrated in FIG. 15, the imaging unit 1d in the fourth example embodiment is different from the imaging unit 1 in the second example embodiment, in that the arithmetic apparatus 17 includes a luminance control unit 175d. The luminance control unit 175d is a specific example of the "luminance control unit" described in Supplementary Note later. The luminance control unit 175d changes luminance of the iris image IMG_I based on the rotation amount (i.e., the driving amount) of the camera base 151 described in the third example embodiment. In this instance, the imaging unit 1 may transmit the iris image IMG_I with the luminance changed, to the authentication server 2. Other features of the imaging unit 1d may be the same as those of the imaging unit 1.

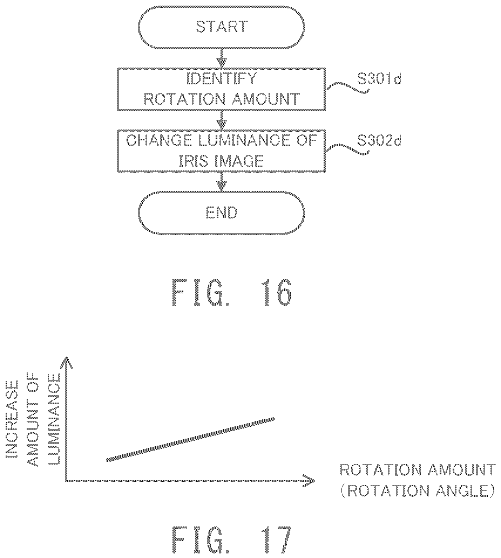

The luminance control unit 175d may change the luminance of the iris image IMG_I in accordance with the flowchart illustrated in FIG. 16, after the iris camera 12 generates the iris image IMG_I by imaging the eye of the target person P in the step S106 in FIG. 9 described above. Specifically, first, the luminance control unit 175d identifies the rotation amount of the camera base 151 by the drive motor 15 (step S301d). For example, as in the third example embodiment, the luminance control unit 175d may identify the rotation amount, by acquiring the information about the rotation amount of the camera base 151 from the rotation control unit 172. Thereafter, the luminance control unit 175d changes the luminance of the iris image IMG_I based on the rotation amount acquired in the step S301d (step S302d).

In the step S302d, for example, the luminance control unit 175d may change the luminance of the iris image IMG_I such that the luminance changed based on the rotation amount falls within a constant luminance range. For example, as described in the third example embodiment, when the opening 195 of the housing 19 is filled with the optical member 1951, as the rotation angle θ is increased, the intensity of the illumination light reaching the target person P is reduced and the intensity of the return light reaching the iris camera 12 is reduced. Therefore, as the rotation angle θ is increased, the luminance of the iris image IMG_I is reduced. Consequently, when the luminance of the iris image IMG_I is excessively low, the authentication unit 211 that authenticates the target person P based on the iris image IMG_I, may not be capable of properly extracting the iris pattern from the iris image IMG_I. Therefore, the luminance control unit 175d may change the luminance of the iris image IMG_I such that the luminance of the iris image IMG_I falls within a constant luminance range in which it is possible to properly extract the iris pattern from the iris image IMG_I. As an example, as illustrated in FIG. 17, the luminance control unit 175d may change (e.g., increase) the luminance of the iris image IMG_I such that, as the rotation angle θ is increased, an increase amount of the luminance of the iris image IMG_I is increased.

Consequently, even when the rotation angle θ is changed, the luminance of the iris image IMG_I is not excessively reduced. Therefore, the authentication unit 211 is capable of properly extracting the iris pattern from the iris image IMG_I. Consequently, the authentication unit 211 is capable of properly authenticating the target person P.

The luminance control unit 175d may change luminance of face image IMG_F, in addition to or in place of changing the luminance of the iris image IMG_I. An aspect of changing the luminance of the face image IMG_F may be the same as that of the iris image IMG_I. In this case, in the step S103 in FIG. 9 described above, the rotation control unit 172 may identify the position of the eye of the target person P (especially, the position in the vertical direction, and the position in the Z-axis direction, for example) based on the face image IMG_F with the luminance changed. Consequently, even when the rotation angle θ is changed, the rotation control unit 172 is capable of properly identifying the position of the eye of the target person P.

In addition, the information processing system SYS4 in the fourth example embodiment may include the constituent components unique to the information processing system SYS3 in the third example. The constituent components unique to the information processing system SYS3 in the third example embodiment may include a constituent component related to the lighting control unit 174c.

### (5) Fifth Example Embodiment

Next, an information processing system and an information processing apparatus in a fifth example embodiment will be described. The following describes an information processing system SYS5 to which the information processing system and the information processing apparatus in the fifth example embodiment are applied. The information processing system SYS5 in the fifth example embodiment is different from the information processing system SYS2 in the second example embodiment in that it includes an imaging unit 1e in place of the imaging unit 1. Other features of the information processing system SYS5 may be the same as those of the information processing system SYS2.

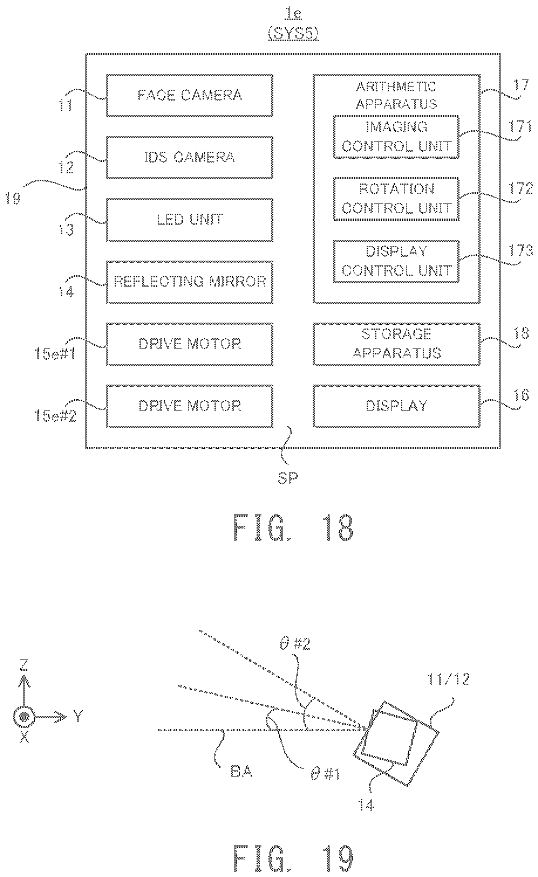

Hereinafter, with reference to FIG. 18, the imaging unit 1e in the fifth example embodiment will be described. FIG. 18 is a block diagram illustrating a configuration of the imaging unit 1e in the fifth example embodiment.

As illustrated in FIG. 18, the imaging unit 1e in the fifth example embodiment is different from the imaging unit 1 in the second example embodiment in that the imaging unit 1e includes a drive motor 15e#1 and a drive motor 15e#2 in place of the drive motor 15. The drive motor 15e#1 is a specific example of the "first drive unit" described in Supplementary Note later. The drive motor 15e#2 is a specific example of the" second drive unit" described in Supplementary Note later. Other features of the imaging unit 1e may be the same as those of the imaging unit 1.

The drive motor 15e#1 is different from the drive motor 15, in that the drive motor 15e#1 rotates (i.e., drives) the reflecting mirror 14, but may not rotate (i.e., may not drive) the face camera 11 and iris camera 12. The drive motor 15e#2 is different from the drive motor 15, in that the drive motor 15e#2 rotates (i.e., drives) the face camera 11 and the iris camera 12, but may not rotate (i.e., may not drive) the reflecting mirror 14. That is, in the fifth example embodiment, each of the face camera 11 and the iris camera 12, and the reflecting mirror 14 are driven by the separate drive motors 15e#1 and 15e#2, respectively. Other features of the drive motors 15e#1 and 15e#2 may be the same as those of the drive motor 15.

A rotation amount (i.e., a driving amount) of the reflecting mirror 14 by the drive motor 15e#1 may be different from a rotation amount (i.e., a driving amount) of each of the face camera 11 and the iris camera 12 by the drive motor 15e#2. For example, as illustrated in FIG. 19, a rotation angle θ#1 of the reflecting mirror 14 by the drive motor 15e#1 may be different from a rotation angle θ#2 of each of the face camera 11 and the iris camera 12 by the drive motor 15e#2. Typically, the rotation amount of the reflecting mirror 14 by the drive motor 15e#1 may be less than the rotation amount of each of the face camera 11 and iris camera 12 by the drive motor 15e#2. For example, the rotation angle θ#1 of the reflecting mirror 14 by the drive motor 15e#1 may be less than the rotation angle θ#2 of each of the face camera 11 and the iris camera 12 by the drive motor 15e#2. As an example, the rotation amount of the reflecting mirror 14 by the drive motor 15e#1 may be half of the rotation amount of each of the face camera 11 and the iris camera 12 by the drive motor 15e#2. For example, the rotation angle θ#1 of the reflecting mirror 14 by the drive motor 15e#1 may be half of the rotation angle θ#2 of each of the face camera 11 and the iris camera 12 by the drive motor 15e#2.

An example of a scene where the face camera 11, the iris camera 12, and the reflecting mirror 14 are rotated such that the rotation amount of the reflecting mirror 14 is different from the rotation amount of each of the face camera 11 and the iris camera 12, is a scene where the optical axis of the LED unit 13 is not coaxial with the rotating shaft 150 of at least one of the drive motors 15e#1 and 15e#2. That is, an example of a scene where the face camera 11, the iris camera 12, and the reflecting mirror 14 are rotated such that the rotation amount of the reflecting mirror 14 is different from the rotation amount of each of the face camera 11 and the iris camera 12, is a scene where the optical axis of the LED unit 13 is not coaxial with the rotation axis of at least one of the face camera 11, the iris camera 12, and the reflecting mirror 14. In such scenes, if it is assumed that the rotation amount of the reflecting mirror 14 is the same as the rotation amount of each of the face camera 11 and the iris camera 12, the return light of the illumination light reflected by the reflecting mirror 14 may not enter the iris camera 12 due to a deviation between the optical axis and the rotation axis of the LED unit 13. Therefore, the imaging unit 1e rotates the face camera 11, the iris camera 12, and the reflecting mirror 14 such that the rotation amount of the reflecting mirror 14 is different from the rotation amount of each of the face camera 11 and the iris camera 12, thereby canceling out an influence of the deviation between the optical axis and the rotation axis of the LED unit 13. Conversely, the imaging unit 1e rotates the face camera 11, the iris camera 12, and the reflecting mirror 14 so as to cancel out the influence of the deviation between the optical axis and the rotation axis of the LED unit 13. Consequently, even when the optical axis and the rotation axis of the LED unit 13 are not coaxial, the return light of the illumination light reflected by the reflecting mirror 14 properly enters the iris camera 12. At this time, the illumination light reflected by the reflecting mirror 14 passes through both an optical path from the imaging unit 1 toward the target person P and an optical path from the target person P toward the imaging unit 1. Therefore, an angular deviation of the illumination light caused by the deviation between the optical axis and the rotation axis of the LED unit 13 is doubled. From this point of view, the rotation amount of the reflecting mirror 14 may be half of the rotation amount of each of the face camera 11 and the iris camera 12.

The rotation amount (i.e., the driving amount) of the reflecting mirror 14 by the drive motor 15e#1, however, may be the same as the rotation amount (i.e., the driving amount) of each of the face camera 11 and the iris camera 12 by the drive motor 15e#2. For example, the rotation angle θ#1 of the reflecting mirror 14 by the drive motor 15e#1 may be the same as the rotation angle θ#2 of each of the face camera 11 and the iris camera 12 by the drive motor 15e#2. As described above, however, when the face camera 11, the iris camera 12 and the reflecting mirror 14 are rotated such that the rotation amount of the reflecting mirror 14 is the same as the rotation amount of each of the face camera 11 and the iris camera 12, the return light of the illumination light reflected by the reflecting mirror 14 may not enter the iris camera 12. Consequently, the iris camera 12 may not be capable of properly imaging the eye of the target person P. Therefore, in a case where the iris camera 12 fails to properly image the eye of the target person P while the face camera 11, the iris camera 12, and the reflecting mirror 14 are rotated such that the rotation amount of the reflecting mirror 14 is the same as the rotation amount of each of the face camera 11 and the iris camera 12, the imaging unit 1e may rotate the face camera 11, the iris camera 12, and the reflecting mirror 14 again such that the rotation amount of the reflecting mirror 14 is different from the rotation amount of each of the face camera 11 and the iris camera 12.

The imaging unit 1e may further include a drive motor 15e#3 that rotates (i.e., drives) the LED unit 13. In this instance, the drive motor 15e#3 may rotate the LED unit 13, in accordance with the rotation of the reflecting mirror 14 by the drive motor 15e#1. The drive motor 15e#3 may rotate the LED unit 13, in the same direction as a direction in which the drive motor 15e#1 rotates the reflecting mirror 14. As an example, when the drive motor 15e#1 rotates the reflecting mirror 14 upward such that the reflecting mirror 14 is directed more upward (i.e., the rotation angle θ#1 is increased), the drive motor 15e#3 may rotate the LED unit 13 upward such that the LED unit 13 emits the illumination light more upward. Consequently, even when the target person P who is very tall is located in front of the imaging unit 1e, the upward-directed LED unit 13 is capable of emitting the illumination light toward the target person P who is very tall, through the upward-directed reflecting mirror 14.

In addition, the information processing system SYS5 in the fifth example embodiment may include the constituent components unique to the information processing system SYS3 in the third example embodiment to the information processing system SYS4 in the fourth example embodiment. The constituent components unique to the information processing system SYS4 in the fourth example embodiment may include a constituent component related to the luminance control unit 175d.

### (6) Sixth Example Embodiment

Next, an information processing system and an information processing apparatus in a sixth example embodiment will be described. The following describes an information processing system SYS6 to which the information processing system and the information processing apparatus in the sixth example embodiment are applied. The information processing system SYS6 in the sixth example embodiment is different from the information processing system SYS2 in the second example embodiment in that it includes an imaging unit 1f in place of the imaging unit 1. Other features of the information processing system SYS6 may be the same as those of the information processing system SYS2.

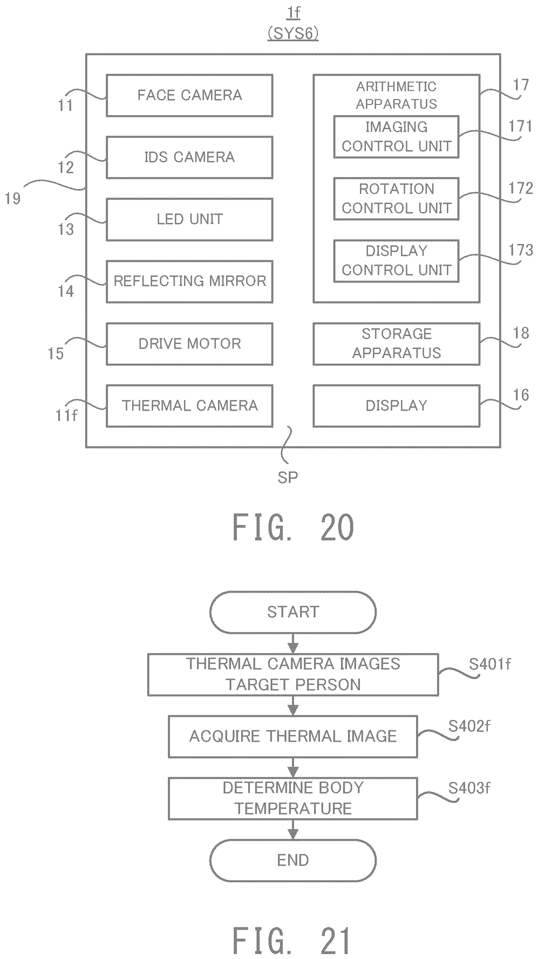

Hereinafter, with reference to FIG. 20, the imaging unit 1f in the sixth example embodiment will be described. FIG. 20 is a block diagram illustrating a configuration of the imaging unit 1f in the sixth example embodiment.

As illustrated in FIG. 20, the imaging unit 1f in the sixth example embodiment is different from the imaging unit 1 in the second example embodiment in that the imaging unit 1f includes a thermal camera 11f. The thermal camera 11f is a specific example of the "third imaging unit" described in Supplementary Note later. Other features of the imaging unit 1f may be the same as those of the imaging unit 1.

The thermal camera 1 1f images the target person P, thereby generating body temperature information indicating a body temperature of the target person P imaged by the thermal camera 11f. The body temperature information may include image information indicating the body temperature of the target person (i.e., a body temperature image, and hereinafter referred to as a "thermal image"). The thermal image may be an image indicating a body temperature distribution of the target person P by color or gradation. The body temperature information may include numerical information quantitatively indicating the body temperature of the target person P. The body temperature information may include any data directly or indirectly indicating the body temperature of the target person P. The following describes an example in which the thermal camera 11f generates the thermal image, for convenience of description.

The authentication unit 211 of the authentication server 2 may perform a body temperature determination operation using thermal image, in addition to or in place of the authentication operation. A flow of the body temperature determination operation is illustrated in FIG. 21. As illustrated in FIG. 21, first, as a prerequisite for the body temperature determination operation, the thermal camera 11f generates the thermal image by imaging the target person P (step S401f). Thereafter, the authentication unit 211 acquires the thermal image from the imaging unit 1f (step S402f). Thereafter, the authentication unit 211 may determine whether or not the body temperature of the target person P is normal based on thermal image acquired in the step S402f.

When it is determined that the body temperature of the target person P is normal, the authentication unit 211 may perform an operation to be performed when the body temperature of the target person P is determined to be normal. For example, as described above, when the information processing system SYS2 is used to manage/control the entry of the target person P to the entry restricted area, the authentication unit 211 may set the state of the gate apparatus disposed at the entrance/exit of the entry restricted area into the opening state in which the gate apparatus does not block the passage of the target person P.

On the other hand, when it is determined that the body temperature of the target person P is not normal, the authentication unit 211 may perform an operation to be performed when the body temperature of the target person P is determined to be not normal. For example, as described above, when the information processing system SYS2 is used to manage/control the entry of the target person P to the entry restricted area, the authentication unit 211 may set the state of the gate apparatus disposed at the entrance/exit of the entry restricted area into the closing state in which the gate apparatus prevents the passage of the target person P.

As described above, the information processing system SYS6 in the sixth example embodiment is capable of performing the operation that takes into account the body temperature of the target person P, by using the thermal image in addition to the iris image IMG I. Therefore, the information processing system SYS6 in the sixth example embodiment is effective for infection control measures.

The drive motor 15 provided in the imaging unit 1f may drive the thermal camera 11f, together with the face camera 11, the iris camera 12, and the reflecting mirror 14. For example, the drive motor 15 may rotate the thermal camera 11f around a predetermined rotation axis, with the face camera 11, the iris camera 12, and the reflecting mirror 14. Alternatively, the imaging unit 1f may include another drive motor for driving (e.g., rotating) the thermal camera 11f in addition to the drive motor 15. A moving aspect of the thermal camera 11f may be the same as a moving aspect of at least one of the face camera 11, the iris camera 12, and the reflecting mirror 14. Therefore, a detailed description of the moving aspect of the thermal camera 11f will be omitted.

The information processing system SYS6 in the sixth example embodiment may include the constituent components unique to the information processing system SYS3 in the third example embodiment to the information processing system SYS5 in the fifth example embodiment. The constituent components unique to the information processing system SYS5 in the fifth example embodiment may include constituent components related to the drive motors 15e#1 and 15e#2.

### (7) Seventh Example embodiment

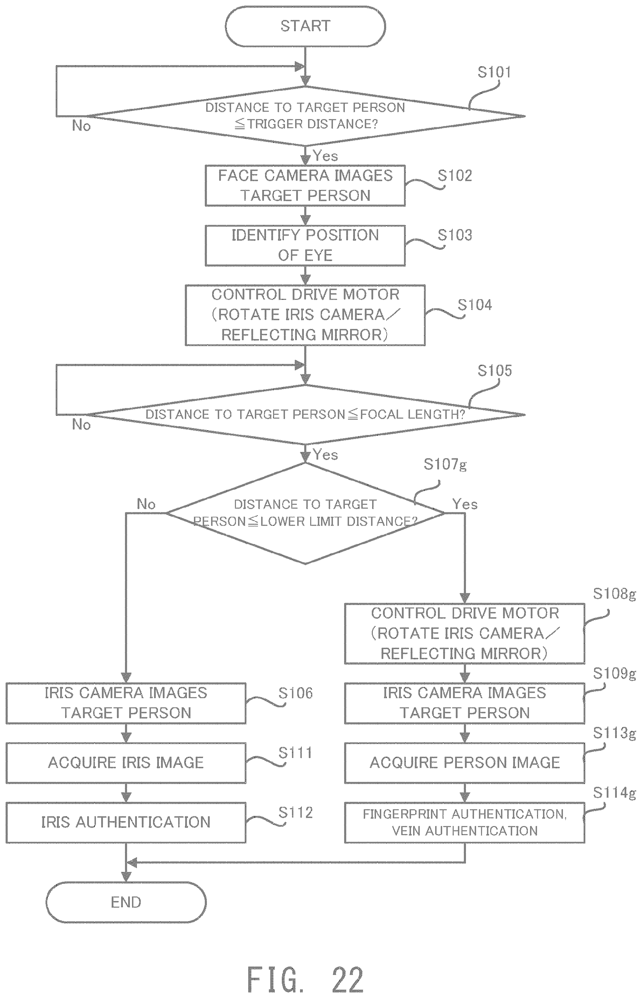

Next, an information processing system and an information processing apparatus in a seventh example embodiment will be described. The following describes an information processing system SYS7 to which the information processing system and the information processing apparatus in the seventh example embodiment are applied. The information processing system SYS7 in the seventh example embodiment is different from the information processing system SYS2 in the second example embodiment in that the flow of the imaging operation and the authentication operation are changed. Other features of the information processing system SYS6 may be the same as those of the information processing system SYS2. Therefore, hereinafter, with reference to FIG. 22, the imaging operation and authentication operation in the seventh example embodiment will be described. FIG. 22 is a flowchart illustrating a flow of the imaging operation and the authentication operation in the seventh example embodiment.

As illustrated in FIG. 22, first, the imaging unit 1 performs the imaging operation (step S101 to step S106 and step S107g to step S109g). Then, the authentication server 2 performs the authentication operation (step S111 to step S112 and step S113g to step S114g).

Specifically, even in the seventh example embodiment, as in the second example embodiment, the imaging unit 1 performs the operation from the step S101 to the step S105.

As a result of the determination in the step S105, when it is determined that the distance from the imaging unit 1 to the target person P is less than or equal to the focal length (the step S105: Yes), the imaging control unit 171 determines whether or not the distance from the imaging unit 1 to the target person P is less than or equal to a predetermined lower limit distance (step S107g). The lower limit distance may be, for example, a threshold that allows distnction between a distance from the imaging unit 1 to the target person P in a state where the iris camera 12 is capable of properly imaging the eye (especially, the iris) of the target person P because the target person P is not too close to the iris camera 12, and a distance from the imaging unit 1 to the target person P in a state where the iris camera 12 is not capable of properly imaging the eye (especially, the iris) of the target person P because the person P is too close to the iris camera 12.

As a result of the determination in the step S107g, when it is determined that the distance from the imaging unit 1 to the target person P is not less than or equal to the predetermined lower limit distance (the step S107g: No), the iris camera 12 is capable of properly imaging the eye (especially, the iris) of the target person P because the target person P is not too close to the iris camera 12. In this instance, the iris camera 12 images the eye (especially the iris) of the target person P (step S106), and the authentication unit 211 authenticates the target person P based on the iris image IMG_I (step S111 to step S112).

On the other hand, as a result of the determination in the step S107g, when it is determined that the distance from the imaging unit 1 to the target person P is less than or equal to the predetermined lower limit distance (the step S107g: Yes), the iris camera 12 may not be capable of properly imaging the eye (especially, the iris) of the target person P because the target person P is too close to the iris camera 12. Therefore, in this case, the iris camera 12 may image a target part that is different from the eye of the target person P (step S109g). In the seventh example embodiment, for example, the iris camera 12 may image a skin of the target person P (step S109g). For example, the iris camera 12 may image the skin of a finger of the target person P (especially, the skin of a fingertip). For example, the iris camera 12 may image the skin of a palm of the target person P. Consequently, the iris camera 12 generates the person image IMG including the target part that is different from the eye of the target person P. For example, the iris camera 12 generates a skin image including the skin of the target person P, as the person image IMG.

Before the iris camera 12 images the target part that is different from the eye of the target person P, the rotation control unit 172 may control the drive motor 15 to rotate the iris camera 12 and the reflecting mirror 14 such that the reflecting mirror 14 is capable of reflecting the illumination light emitted from the LED unit 13 toward the target part of the target person P imaged in the step S109g and such that the iris camera 12 is capable of receiving the return light from the target part of the target person P illuminated with the illumination light (step S108g). As an example, the rotation control unit 172 may control the drive motor 15 to rotate the iris camera 12 and the reflecting mirror 14 such that the reflecting mirror 14 is capable of reflecting the illumination light emitted from the LED unit 13 toward the fingertip of the target person P imaged in the step S109g and such that the iris camera 12 is capable of receiving the return light from the fingertip of the target person P illuminated with the illumination light.

Thereafter, the authentication unit 211 of the authentication server 2 acquires the person image IMG from the iris camera 12 through the communication apparatus 23 (step S113g). That is, the authentication unit 211 acquires the person image IMG including the target part that is different from the eye of the target person P (step S113g). Thereafter, the authentication unit 211 authenticates the target person P based on the person image IMG acquired in the step S113g (step S114g). For example, when the fingertip of the target person P is captured in the person image IMG, the authentication unit 211 may perform fingerprint authentication of authenticating the target person P based on a fingerprint pattern of the fingertip of the target person P. For example, when the palm of the target person P is captured in the person image IMG, the authentication unit 211 may perform palmprint authentication of authenticating the target person P based on a palmprint pattern of a hand of the target person P. For example, when the fingertip or palm of the target person P is captured in the person image IMG, the authentication unit 211 may perform vein authentication of authenticating the target person P based on a vein pattern of the fingertip or palm of the target person P.

As described above, the information processing system SYS7 in the seventh example embodiment is capable of authenticating the target person P even when the iris camera 12 is not capable of imaging the eye (especially, the iris) of the target person P.

The authentication unit 211 of the authentication server 2 may authenticate the target person P based on the iris authentication IMG_I and may authenticate the target person P based on the person image IMG including the target part that is different from the eye of the target person P. That is, the authentication server 2 may function as an authentication apparatus having a multi-modal function.

Furthermore, the iris camera 12 may image the target part that is different from the eye of the target person P even when it is determined that the distance from the imaging unit 1 to the target person P is not less than or equal to the predetermined lower limit distance. For example, when the iris pattern cannot be extracted from the iris image IMG_I generated by the iris camera 12 imaging the eye of the target person P, the iris camera 12 may image the target part that is different from the eye of the target person P. Consequently, even when the iris pattern cannot be extracted from the iris image IMG_I, the authentication server 2 is capable of authenticating the target person P. When surgery is performed on the eye of the target person P or the target person P is wearing color contact lenses, it is more likely that the iris pattern cannot be extracted from the iris image IMG_I. Therefore, in the seventh example embodiment, the authentication server 2 is capable of authenticating the target person P even when surgery is performed on the eye of the target person P or the target person P is wearing color contact lenses.

In addition, as described in the modified examples of the second example embodiment, the authentication server 2 may determine whether or not the target person P makes a predetermined gesture in order to confirm the intention of the target person P. Here, when a gesture of raising a finger (e.g., a thumb-up) is used as the predetermined gesture, the iris camera 12 may image the fingertip of the target person P while the target person P is raising the finger. Consequently, the authentication server 2 is capable of relatively easily acquiring the person image IMG including the fingertip of the target person P (especially, the person image IMG usable for the fingerprint authentication or the vein authenticate described above), thereby authenticating the target person P.

In the above description, the iris camera 12 images the target part (e.g., the skin) that is different from the eye of the target person P. However, in addition to or in place of the iris camera 12, the face camera 11 may image the target part (e.g., the skin) that is different from the eye of the target person P.

### (8) Supplementary Notes

With respect to the example embodiments described above, the following Supplementary Notes are further disclosed.

### [Supplementary Note 1]

An information processing apparatus including:
a first imaging unit that is configured to image a target;
a second imaging unit that is configured to image the target;
a lighting unit that is configured to emit illumination light;
a reflection unit that reflects the illumination light emitted by the lighting unit toward the target; and
a drive unit that is disposed at a different position from a position where the lighting unit is disposed, and that drives each of the first imaging unit, the second imaging unit, and the reflection unit.

### [Supplementary Note 2]

The Information processing apparatus according to Supplementary Note 1, wherein
the information processing apparatus further includes a housing that accommodates therein the first imaging unit, the second imaging unit, the lighting unit, the reflection unit, and the drive unit, and
at least a part of the housing functions as a heat radiating member capable of radiating a heat of an inside of the housing to an outside of the housing.

### [Supplementary Note 3]

The information processing apparatus according to Supplementary Note 1 or 2, wherein
the first imaging unit generates a face image by imaging a face of the target,
the second imaging unit generates an iris image by imaging an iris of the target, and
the information processing apparatus further includes a drive control unit that identifies a position of an eye of the target based on the face image and that controls the drive unit such that the reflection unit reflects the illumination light toward the identified position of the eye.

### [Supplementary Note 4]

The information processing apparatus according to any one of Supplementary Notes 1 to 3, wherein the drive unit drives each of the first imaging unit, the second imaging unit, and the reflection unit such that each of the first imaging unit, the second imaging unit, and the reflection unit is rotated around a predetermined rotation axis.

### [Supplementary Note 5]

The information processing apparatus according to any one of Supplementary Notes 1 to 4, further including an intensity control unit that changes intensity of the illumination light emitted by the lighting unit, based on respective driving amounts of the first imaging unit, the second imaging unit, and the reflection unit, by the drive unit.

### [Supplementary Note 6]

The information processing apparatus according to any one of Supplementary Notes 1 to 5, wherein
the first imaging unit generates a face image by imaging a face of the target,
the second imaging unit generates an iris image by imaging an iris of the target, and
the information processing apparatus further comprises a luminance control unit that changes at least one of luminance of the face image captured by the first imaging unit and luminance of the iris image captured by the second imaging unit, based on respective driving amounts of the first imaging unit, the second imaging unit, and the reflection unit, by the drive unit.

### [Supplementary Note 7]

The Information processing apparatus according to any one of Supplementary Notes 1 to 6, wherein
the drive unit drives each of the first imaging unit, the second imaging unit, and the reflection unit such that each of the first imaging unit, the second imaging unit, and the reflection unit is rotated around a predetermined rotation axis, and
an optical axis of the lighting unit is coaxial with the rotation axis.

### [Supplementary Note 8]

The information processing apparatus according to any one of Supplementary Notes 1 to 7, wherein
the drive unit includes:
a first drive unit that drives the reflection unit such that the reflection unit is rotated around a predetermined rotation axis; and
a second drive unit that drives each of the first imaging unit and the second imaging unit such that each of the first imaging unit and the second imaging unit is rotated around the rotational axis, and
a rotation amount of the reflection unit by the first drive unit is different from a rotation amount of each of the first imaging unit and the second imaging unit by the second drive unit.

### [Supplementary Note 9]

The information processing apparatus according to any one of Supplementary Notes 1 to 8, wherein the information processing apparatus comprises a third imaging unit that is configured to generate body temperature information indicating a body temperature of the target, by imaging the target.

### [Supplementary Note 10]

The information processing apparatus according to Supplementary Note 9, wherein the drive unit drives the third imaging unit.

### [Supplementary Note 11]

The information processing apparatus according to any one of Supplementary Notes 1 to 10, wherein
the first imaging unit generates a face image by imaging a face of the target,
the drive unit drives the reflection unit and the second imaging unit such that the reflection unit reflects the illumination light toward an iris of the target and such that the second imaging unit generates an iris image by imaging the iris of the target when a distance between the second imaging unit and the target exceeds a predetermined threshold, and drives the reflection unit and the second imaging unit such that the reflection unit reflects the illumination light toward a fingerprint of the target and such that the second imaging unit generates a skin image by imaging a skin of the target when the distance falls below the predetermined threshold, and
at least one of the face image, the iris image, and the skin image is used to authenticate the target.

### [Supplementary Note 12]

An information processing system including:
an information processing apparatus; and
an authentication apparatus, wherein
the information processing apparatus includes:
   a first imaging unit that is configured to generate a first image by imaging a target;
   a second imaging unit that is configured to generate a second image by imaging the target;
   a lighting unit that is configured to emit illumination light;
   a reflection unit that reflects the illumination light emitted by the lighting unit toward the target; and
   a drive unit that is disposed at a different position from a position where the lighting unit is disposed, and that drives each of the first imaging unit, the second imaging unit, and the reflection unit, and
   the authentication apparatus includes an authenticate unit that authenticates the target by using at least one of the first image and the second image.

At least a part of the constituent components of each of the example embodiments described above can be combined with at least another part of the constituent components of each of the example embodiments described above, as appropriate. A part of the constituent components of each of the example embodiments described above may not be used. Furthermore, to the extent permitted by law, all the references (e.g., publications) cited in this disclosure are incorporated by reference as a part of the description of this disclosure.

This disclosure is allowed to be changed, if desired, without departing from the essence or spirit of this disclosure which can be read from the claims and the entire identification. An information processing apparatus and an information processing system with such changes are also intended to be within the technical scope of this disclosure.

### Description of Reference Codes

1 Imaging unit
11 Face camera
12 Iris camera
13 The LED unit
14 Reflecting mirror
15 Drive motor
17 Arithmetic apparatus
171 Imaging control unit
172 Rotation control unit
173 Display control unit
19 Housing
2 Authentication server
21 Arithmetic apparatus
211 Authentication unit
SYS1 to SYS7 Information processing system
P The target person
IMG_F Face image
IMG_I Iris image

## Claims

1. An information processing apparatus comprising:
a first imaging unit that is configured to image a target;
a second imaging unit that is configured to image the target;
a lighting unit that is configured to emit illumination light;
a reflection unit that reflects the illumination light emitted by the lighting unit toward the target; and
a drive unit that is disposed at a different position from a position where the lighting unit is disposed, and that drives each of the first imaging unit, the second imaging unit, and the reflection unit.

2. The Information processing apparatus according to claim 1, wherein
the information processing apparatus further comprises a housing that accommodates therein the first imaging unit, the second imaging unit, the lighting unit, the reflection unit, and the drive unit, and
at least a part of the housing functions as a heat radiating member capable of radiating a heat of an inside of the housing to an outside of the housing.

3. The information processing apparatus according to claim 1 or 2, wherein
the first imaging unit generates a face image by imaging a face of the target,
the second imaging unit generates an iris image by imaging an iris of the target, and
the information processing apparatus further comprises a drive control unit that identifies a position of an eye of the target based on the face image and that controls the drive unit such that the reflection unit reflects the illumination light toward the identified position of the eye.

4. The information processing apparatus according to any one of claims 1 to 3, wherein the drive unit drives each of the first imaging unit, the second imaging unit, and the reflection unit such that each of the first imaging unit, the second imaging unit, and the reflection unit is rotated around a predetermined rotation axis.

5. The information processing apparatus according to any one of claims 1 to 4, further comprising an intensity control unit that changes intensity of the illumination light emitted by the lighting unit, based on respective driving amounts of the first imaging unit, the second imaging unit, and the reflection unit, by the drive unit.

6. The information processing apparatus according to any one of claims 1 to 5, wherein
the first imaging unit generates a face image by imaging a face of the target,
the second imaging unit generates an iris image by imaging an iris of the target, and
the information processing apparatus further comprises a luminance control unit that changes at least one of luminance of the face image captured by the first imaging unit and luminance of the iris image captured by the second imaging unit, based on respective driving amounts of the first imaging unit, the second imaging unit, and the reflection unit, by the drive unit.

7. The Information processing apparatus according to any one of claims 1 to 6, wherein
the drive unit drives each of the first imaging unit, the second imaging unit, and the reflection unit such that each of the first imaging unit, the second imaging unit, and the reflection unit is rotated around a predetermined rotation axis, and
an optical axis of the lighting unit is coaxial with the rotation axis.

8. The information processing apparatus according to any one of claims 1 to 7, wherein
the drive unit includes:
a first drive unit that drives the reflection unit such that the reflection unit is rotated around a predetermined rotation axis; and
a second drive unit that drives each of the first imaging unit and the second imaging unit such that each of the first imaging unit and the second imaging unit is rotated around the rotational axis, and
a rotation amount of the reflection unit by the first drive unit is different from a rotation amount of each of the first imaging unit and the second imaging unit by the second drive unit.

9. The information processing apparatus according to any one of claims 1 to 8, wherein the information processing apparatus comprises a third imaging unit that is configured to generate body temperature information indicating a body temperature of the target, by imaging the target.

10. The information processing apparatus according to claim 9, wherein the drive unit drives the third imaging unit.

11. The information processing apparatus according to any one of claims 1 to 10, wherein
the first imaging unit generates a face image by imaging a face of the target,
the drive unit drives the reflection unit and the second imaging unit such that the reflection unit reflects the illumination light toward an iris of the target and such that the second imaging unit generates an iris image by imaging the iris of the target when a distance between the second imaging unit and the target exceeds a predetermined threshold, and drives the reflection unit and the second imaging unit such that the reflection unit reflects the illumination light toward a fingerprint of the target and such that the second imaging unit generates a skin image by imaging a skin of the target when the distance falls below the predetermined threshold, and
at least one of the face image, the iris image, and the skin image is used to authenticate the target.

12. An information processing system comprising:
an information processing apparatus; and
an authentication apparatus, wherein
the information processing apparatus includes:
a first imaging unit that is configured to generate a first image by imaging a target;
a second imaging unit that is configured to generate a second image by imaging the target;
a lighting unit that is configured to emit illumination light;
a reflection unit that reflects the illumination light emitted by the lighting unit toward the target; and
a drive unit that is disposed at a different position from a position where the lighting unit is disposed, and that drives each of the first imaging unit, the second imaging unit, and the reflection unit, and
the authentication apparatus includes an authenticate unit that authenticates the target by using at least one of the first image and the second image.
